# EUROPEAN PATENT SPECIFICATION

(11) **EP 2 147 107 B1**
(45) Date of publication and mention of the grant of the patent: **20.07.2011**
(21) Application number: 08750156.5
(22) Date of filing: 07.05.2008
(51) Int. Cl.: C12N 15/81, C12R 1/685

(54) **EXPRESSION CLONING METHOD SUITABLE FOR SELECTING LIBRARY CLONES PRODUCING A POLYPEPTIDE OF INTEREST**
EXPRESSIONS-CLONING-VERFAHREN ZUR AUSWAHL VON BIBLIOTHEKSKLONEN ZUR HERSTELLUNG EINES POLYPEPTIDS VON INTERESSE
PROCÉDÉ DE CLONAGE D'EXPRESSION, APPROPRIÉ POUR SÉLECTIONNER DES CLONES DE BIBLIOTHÈQUE PRODUISANT UN POLYPEPTIDE D'INTÉRÊT

(30) Priority: 09.05.2007 DK 200700694
(43) Date of publication of application: 27.01.2010
(73) Proprietor: Novozymes A/S, 2880 Bagsvaerd (DK)
(72) Inventor: VIND, Jesper, DK-3500 Vaerloese (DK)
(86) International application number: PCT/EP2008/055638
(87) International publication number: WO 2008/138835

(56) References cited:
- WO-A-00/24883
- WO-A-01/68882
- WO-A-03/070956
- WO-A-2005/095624
- WO-A2-02/052026
- MALAVAZI IRAN ET AL: "THE ASPERGILLUS NIDULANS SLDI(RAD50) GENE INTERACTS WITH BIME(APC1), A HOMOLOGUE OF AN ANAPHASE-PROMOTING COMPLEX SUBUNIT" MOLECULAR MICROBIOLOGY, BLACKWELL SCIENTIFIC, OXFORD, GB, vol. 57, no. 1, July 2005 (2005-07), pages 222-237, XP008076668 ISSN: 0950-382X
- ALEKSENKO A Y ET AL: "Recombinational stability of replicating plasmids in Aspergillus nidulans during transformation, vegetative growth and sexual reproduction" CURRENT GENETICS, vol. 28, no. 1, 1995, pages 87-93, XP002457614 ISSN: 0172-8083

## Description

### Sequence listing

The present invention comprises a sequence listing.

### FIELD OF THE INVENTION

The present invention relates to an expression cloning method suitable for selecting library clones producing a polypeptide of interest.

### BACKGROUND OF THE INVENTION

Several methods for the construction of libraries of polynucleotide sequences of interest in yeast have been disclosed in which the libraries are screened in yeast prior to transformation of an industrially relevant filamentous fungal host cell with a selected polynucleotide.

Often however, a polynucleotide sequence identified by screening in yeast or bacteria cannot be expressed or is expressed at low levels when transformed into production relevant filamentous fungal cells. This may be due to any number of reasons, including differences in codon usage, regulation of mRNA levels, translocation apparatus, post-translational modification machinery (e.g., cysteine bridges, glycosylation and acylation patterns), etc.

A. Aleksenko and A.J. Clutterbuck (1997. Fungal Genetics and Biology 21:373-387) disclose the use of autonomous replicative vectors, or autonomously replicating sequences (ARS), for gene cloning and expression studies. AMA1 (autonomous maintenance in *Aspergillus*) is one of the plasmid replicator elements discussed. It consists of two inverted copies of a genomic repeat designated MATE1 (mobile *Aspergillus* transformation enhancer) separated by a 0.3 kb central spacer. AMA1 promotes plasmid replication with little rearrangement, multimerization or chromosomal integration. AMA1-based plasmids provide two advantages in gene cloning and library generation in filamentous fungi. The first is a high frequency of transformation which both increases the potential library size. Secondly, by providing a reasonably stable and standard environment for gene expression, the properties of the transformants will be uniform (WO 00/24883; Novozymes A/S).

In a study of cellular response systems to DNA damage using *A. nidulans* as a model organism I. Malavazi et al., Microbial Microbiology, 57(1): 222-237 used a method involving transforming a *sldl1444D* mutant with a ligation mixture comprising an AMA1 based vector and genomic DNA fragments in order to isolate a gene involved in said system. The sldl gene was identified as the *A. nidulans* a homologous of the Rad50 gene.

WO 94/11523 and WO 01/51646 disclose expression vectors comprising a fully impaired consensus Kozak or "crippled" consensus Kozak sequence.

WO 03/070956 discloses a cloning vector for expression cloning comprising the AMA1-sequence and a crippled translation initiation sequence.

Expression cloning as such in filamentous fungi is presently part of the standard methodology in the art, however the use of such methods is of such industrial relevance that even minor increments in efficiency, performance or economy is of great interest.

### SUMMARY OF THE INVENTION

It is desirable to screen a polynucleotide library for a polypeptide with a property of interest in a filamentous fungal host cell in a manner which allows quick and easy characterization of the subsequent polypeptide. The method described in WO 03/070956 has now been further improved by providing reduced variation in copy number of the expression vector after transformation into the expression host cell. This has according to the present invention been achieved by decreasing the frequency of non-homologous recombination of the expression plasmid into the genome of the host cell.

An aspect of the present invention relates to methods for producing a recombinant polypeptide of interest, the method comprising the steps of:
a) providing a polynucleotide library encoding one or more polypeptides of interest, wherein the library was prepared in an expression cloning vector comprising at least the following elements:
   i) a polynucleotide encoding a selectable marker;
   ii) a fungal replication initiation sequence, preferably an autonomously replicating sequence (ARS); and
   iii) a polynucleotide comprising in sequential order: a promoter derived from a fungal cell, a cloning-site into which the library is cloned, and a transcription terminator;
b) transforming a mutant of a parent filamentous fungal host cell with the library, wherein the frequency of non-homologous recombination in the mutant has been decreased compared to the parent;
c) culturing the transformed host cell obtained in (b) under conditions suitable for expression of the polynucleotide library;
d) selecting a transformed host cell which produces the polypeptide of interest; and
e) recovering the polypeptide of interest.

### DETAILED DESCRIPTION OF THE INVENTION

According to the present invention it has been discovered that one potential problem when employing expression cloning methods is an uneven expression level after transformation of libraries into expression vectors making it more difficult to compare the results of individual clones and consequently making the selection process more difficult. Several factors could be responsible for the observed uneven expression levels. Previously this has been addressed by improving the components making up the expression vector as described above, e.g. by incorporating the AMA1-sequences into the vector or by using a crippled "consensus" Kozak sequence. According to the present invention it has now been discovered that a significant improvement can be obtained by decreasing non-homologous recombination frequency in the expression host cell.

In one aspect the invention therefore relates to a method for producing a recombinant polypeptide of interest, the method comprising the steps of:
a) providing a polynucleotide library encoding one or more polypeptides of interest, wherein the library was prepared in an expression cloning vector comprising at least the following elements:
   i) a polynucleotide encoding a selectable marker;
   ii) a fungal replication initiation sequence, preferably an autonomously replicating sequence (ARS); and
   iii) a polynucleotide comprising in sequential order: a promoter derived from a fungal cell, a cloning-site into which the library is cloned, and a transcription terminator;
b) transforming a mutant of a parent filamentous fungal host cell with the library, wherein the frequency of non-homologous recombination in the mutant has been decreased compared to the parent;
c) culturing the transformed host cell obtained in (b) under conditions suitable for expression of the polynucleotide library;
d) selecting a transformed host cell which produces the polypeptide of interest; and
e) recovering the polypeptide of interest.

After selection of the transformed host cell the polynucleotide encoding the polypeptide of interest is isolated from the host cell of step (d) in order to identify mutations and subsequently retransform the variant gene into a clean host cell to ensure that no cross contamination from other variants have taken place.

In the production methods of the present invention, the cells are cultivated in a nutrient medium suitable for production of the polypeptide, and under conditions that select for multiple copies of the selectable marker, using methods known in the art. For example, the cell may be cultivated in 24, 96, 384 or 1536 well microtiter plates, by shake flask cultivation, or small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection).

If the polypeptide of interest is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods may include use of specific antibodies, formation of an enzyme product, or disappearance of an enzyme substrate. The polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides may be purified by a variety of procedures known in the art including, but not limited to, chromatography (*e.g*., ion exchange, affinity, hydrophobic, chromatofocusing, and size exclusion), electrophoretic procedures (*e.g*., preparative isoelectric focusing), differential solubility (*e.g*., ammonium sulfate precipitation), SDS-PAGE, or extraction (see, *e.g*., Protein Purification, J.-C. Janson and Lars Ryden, editors, VCH Publishers, New York, 1989).

### Non-homologous recombination

In Eukaryotes integration into the genome by recombination can occur by two different pathways; one via homologous recombination (HR) and one by non-homologous recombination (NHR). In yeast the preferred pathway is HR while in filamentous fungi most integration events occur by NHR. WO 02/052026 discloses mutants of *Saccharomyces cerevisiae* having an improved targeting efficiency of DNA sequences into its genome. These mutants are deficient in KU70 involved in NHR. Mammalian cells deficient in KU70 have been isolated (Pierce et al. Genes and Development, 2001, 15: 3237-3242), however, such mutants do not display the same phenotype of increased homology-directed targeted integration. In the filamentous fungus *Aspergillus niger* mutants in KU70 resulted in an improved efficiency for targeted integration into the genome (WO 2005/095624). From yeast studies several genes have been implicated in the NHR pathway: KU70, KU80, RAD50, MRE11, XRS2, LIG4 and SIR4 (van den Bosch et al., 2002, Biol. Chem. 383: 873-892 and Allen et al., 2003, Mol. Cancer Res. 1: 913-920). See also table 2, page 23 in WO 02/052026. Functional equivalents of these genes have also been identified in filamentous fungi, WO 2005/095624, which describes the KU70 and KU80 homologues *hdfA* and *hdfB* from *Aspergillus niger,* and Ishibashi, K., Suzuki, K., Ando, Y., Takakura, C., and Inoue, H., 2006, PNAS, USA. 103(40): 14871-14876, which discloses MUS-53 (a LIG4 homologue) in Neurospora.

Particularly the host filamentous fungal cell according to the invention is a mutant resulting in a decrease in frequency of non-homologous recombination. Components involved in NHR comprise filamentous fungal functional equivalents of the yeast KU70, KU80, RAD50, MREII, XRS2, LIG4, or SIR4, or associating components.

Because the nomenclature of genes differs between organisms a functional equivalent or a functional and/or a functional fragment thereof, are all defined herein as being capable of performing (in function, not in amount) at least one function of the yeast genes KU70, KU80, RAD50, MREII, XRS2, LIG4, or SIR4. Functional equivalents of some of these genes have already been identified in filamentous fungi, particularly in *Aspergillus* (hdfA and hdfB) and Neurospora (MUS-53) as described above. Thus in one embodiment the mutant filamentous fungal host has a reduced or no expression or is deficient in at least one of its endogenous genes which are functional equivalents of one of the yeast genes involved in the NHR pathway selected from the group consisting of KU70, KU80, RAD50, MRE11, XRS2, LIG4 and SIR4. This also includes variants of the same genes, which leads to an inactive or less active protein. More particularly the genes are selected form the group consisting of KU70 and KU80, and in particular the functional equivalents from *Aspergillus oryzae* or *Aspergillus niger.* In one embodiment the genes are hdfA and hdfB or homologues thereof.

In a preferred aspect, the KU70 equivalent comprises a nucleotide sequence having at least 70%, preferably at least 75%, more preferably at least 80%, even more preferably at least 85%, most preferably at least 90%, and even most preferably at least 95% identity to SEQ ID NO: 14. In a most preferred aspect, the KU70 equivalent comprises the nucleotide sequence of SEQ ID NO: 14. In another most preferred aspect, the KU70 equivalent consists of the nucleotide sequence of SEQ ID NO: 14.

In another preferred aspect, the KU80 equivalent comprises a nucleotide sequence having at least 70%, preferably at least 75%, more preferably at least 80%, even more preferably at least 85%, most preferably at least 90%, and even most preferably at least 95% identity to SEQ ID NO: 15. In a most preferred aspect, the KU70 equivalent comprises the nucleotide sequence of SEQ ID NO: 15. In another most preferred aspect, the KU70 equivalent consists of the nucleotide sequence of SEQ ID NO: 15.

In another preferred aspect, the KU70 equivalent comprises a nucleotide sequence having at least 70%, preferably at least 75%, more preferably at least 80%, even more preferably at least 85%, most preferably at least 90%, and even most preferably at least 95% identity to SEQ ID NO: 16. In a most preferred aspect, the KU70 equivalent comprises the nucleotide sequence of SEQ ID NO: 16. In another most preferred aspect, the KU70 equivalent consists of the nucleotide sequence of SEQ ID NO: 16.

In another preferred aspect, the KU80 equivalent comprises a nucleotide sequence having at least 70%, preferably at least 75%, more preferably at least 80%, even more preferably at least 85%, most preferably at least 90%, and even most preferably at least 95% identity to SEQ ID NO: 17. In a most preferred aspect, the KU70 equivalent comprises the nucleotide sequence of SEQ ID NO: 17. In another most preferred aspect, the KU70 equivalent consists of the nucleotide sequence of SEQ ID NO: 17.

According to the present invention the library is cloned into the expression cloning vector and subsequently transformed into a mutant filamentous fungus, which mutant is modified in at least one endogenous gene involved in the NHR pathway resulting in a decrease in the frequency of non-homologous recombination in the mutant compared to the parent filamentous fungus. This decrease in frequency of NHR has now been shown to significantly improve the stability of the expression clones and result in more uniform expression levels ensuring a more reliable selection of interesting clones. The term "modification" is defined herein as an introduction, substitution, or removal of one or more nucleotides in a gene or a regulatory element required for the transcription or translation thereof, as well as a gene disruption, gene conversion, gene deletion, or random or specific mutagenesis of at least one endogenous gene involved in the NHR pathway. The deletion of such gene(s) may be partial or complete. The modification results in a decrease or elimination in expression of at least one endogenous gene involved in the NHR pathway.

In a preferred aspect, the modification results in a decrease or elimination in expression of at least one of the genes selected from the group consisting of KU70, KU80, RAD50, MREII, XRS2, LIG4, or SIR4 or a functional equivalent thereof. Such modification results in a deficient filamentous fungal host cell.

The term "deficient" is defined herein as an filamentous fungal mutant strain which produces no detectable amount of the gene product involved in the NHR pathway compared to the parent filamentous fungal strain when cultivated under identical conditions, or, in the alternative, produces preferably at least 25% less, more preferably at least 50% less, even more preferably at least 75% less, and most preferably at least 95% less compared to the parent filamentous fungal strain when cultivated under identical conditions. The level of gene product produced by a filamentous fungal mutant strain of the present invention may be determined using methods described herein or known in the art.

The "deficient" filamentous fungal mutant strain may be constructed by reducing or eliminating expression of a gene involved in the NHR pathway using methods well known in the art, for example, insertions, disruptions, replacements, or deletions. The portion of the gene to be modified or inactivated may be, for example, the coding region or a regulatory element required for expression of the coding region. An example of such a regulatory or control sequence of a gene may be a promoter sequence or a functional part thereof, i.e., a part which is sufficient for affecting expression of the gene. Other control sequences for possible modification include, but are not limited to, a leader, propeptide sequence, signal sequence, transcription terminator, and transcriptional activator.

The filamentous fungal mutant strains may be constructed by gene deletion techniques to eliminate or reduce the expression of at least one gene involved in the NHR pathway. Gene deletion techniques enable the partial or complete removal of the gene(s) thereby eliminating their expression. In such methods, the deletion of the gene(s) may be accomplished by homologous recombination using a plasmid that has been constructed to contiguously contain the 5' and 3' regions flanking the gene. A preferred strategy for down regulating the expression of a given DNA sequence comprises the deletion of the wild type DNA sequence and/or replacement by a modified DNA sequence, whose expression product is not functional. The deletion and the replacement are preferably performed by the gene replacement technique described in EP 0 357 127 B1.

The filamentous fungal mutant strains may also be constructed by introducing, substituting, and/or removing one or more nucleotides in the gene or a regulatory element thereof required for the transcription or translation thereof. For example, nucleotides may be inserted or removed so as to result in the introduction of a stop codon, the removal of the start codon, or a frame-shift of the open reading frame. Such a modification may be accomplished by site-directed mutagenesis or PCR generated mutagenesis in accordance with methods known in the art. See, for example, Botstein and Shortle, 1985, Science 229: 4719; Lo et al., 1985, Proceedings of the National Academy of Sciences USA 81: 2285; Higuchi et al., 1988, Nucleic Acids Research 16: 7351; Shimada, 1996, Meth. Mol. Biol. 57: 157; Ho et al., 1989, Gene 77: 61; Horton et al., 1989, Gene 77: 61; and Sarkar and Sommer, 1990, BioTechniques 8: 404.

The filamentous fungal mutant strains may also be constructed by gene disruption techniques by inserting into the gene of interest an integrative plasmid containing a nucleic acid fragment homologous to the gene which will create a duplication of the region of homology and incorporate vector DNA between the duplicated regions. Such gene disruption can eliminate gene expression if the inserted vector separates the promoter of the gene from the coding region or interrupts the coding sequence such that a non-functional gene product results. A disrupting construct may be simply a selectable marker gene accompanied by 5' and 3' regions homologous to the gene. The selectable marker enables identification of transformants containing the disrupted gene.

The filamentous fungal mutant strains may also be constructed by the process of gene conversion (see, for example, Iglesias and Trautner, 1983, Molecular General Genetics 189: 73-76). For example, in the gene conversion method, a nucleotide sequence corresponding to the gene(s) is mutagenized *in vitro* to produce a defective nucleotide sequence which is then transformed into the parent filamentous fungal strain to produce a defective gene. By homologous recombination, the defective nucleotide sequence replaces the endogenous gene. It may be desirable that the defective gene or gene fragment also comprises a marker which may be used for selection of transformants containing the defective gene.

The filamentous fungal mutant strains may also be constructed by established anti-sense techniques using a nucleotide sequence complementary to the nucleotide sequence of the gene (Parish and Stoker, 1997, FEMS Microbiology Letters 154: 151-157). More specifically, expression of the gene by a filamentous fungal strain may be reduced or eliminated by introducing a nucleotide sequence complementary to the nucleotide sequence of the gene, which may be transcribed in the strain and is capable of hybridizing to the mRNA produced in the strain. Under conditions allowing the complementary anti-sense nucleotide sequence to hybridize to the mRNA, the amount of protein translated is thus reduced or eliminated.

The filamentous fungal mutant strains may be further constructed by random or specific mutagenesis using methods well known in the art, including, but not limited to, chemical mutagenesis (see, for example, Hopwood, The Isolation of Mutants in Methods in Microbiology (J.R. Norris and D.W. Ribbons, eds.) pp 363-433, Academic Press, New York, 1970) and transposition (see, for example, Youngman et al., 1983, Proc. Natl. Acad. Sci. USA 80: 2305-2309). Modification of the gene may be performed by subjecting the parent strain to mutagenesis and screening for mutant strains in which expression of the gene has been reduced or eliminated. The mutagenesis, which may be specific or random, may be performed, for example, by use of a suitable physical or chemical mutagenizing agent, use of a suitable oligonucleotide, or subjecting the DNA sequence to PCR generated mutagenesis. Furthermore, the mutagenesis may be performed by use of any combination of these mutagenizing methods.

Examples of a physical or chemical mutagenizing agent suitable for the present purpose include ultraviolet (UV) irradiation, hydroxylamine, N-methyl-N'-nitro-N-nitrosoguanidine (MNNG), N-methyl-N'-nitrosogaunidine (NTG) O-methyl hydroxylamine, nitrous acid, ethyl methane sulphonate (EMS), sodium bisulphite, formic acid, and nucleotide analogues. When such agents are used, the mutagenesis is typically performed by incubating the parent strain to be mutagenized in the presence of the mutagenizing agent of choice under suitable conditions, and selecting for mutants exhibiting reduced or no expression of a gene.

### Determination of recombination frequency

Determination of recombination frequency can be used in order to determine if the ratio of NHR/HR has changed in the mutant compared to the wild type. NHR efficiency may be determined essentially as described in Example 4 and alternatively as described in WO 2005/095624 page 6-7.

### Library

The library according to the invention encodes the polypeptide of interest which may be native or heterologous to the filamentous fungal host cell. The polynucleotide encoding the polypeptide of interest may originate from any organism capable of producing the polypeptide of interest, including multicellular organisms and microorganisms e.g. bacteria and fungi. The origin of the polynucleotide may also be synthetic meaning that the library could be comprised of e.g. codon optimized variants encoding the same polypeptide or the library could comprise variants obtained by shuffling techniques known in the art.

### Fungal replication initiating sequences

As used herein, the term "fungal replication initiating sequence" is defined as a nucleic acid sequence which is capable of supporting autonomous replication of an extrachromosomal molecule, e.g., a DNA vector such as a plasmid, in a filamentous fungal host cell, normally without structural rearrangement of the DNA-vector or integration into the host cell genome. The replication initiating sequence may be of any origin as long as it is capable of mediating replication intiating activity in a fungal cell. For instance the replication initiating sequence may be a telomer of human origin which confer to the plasmid the ability to replicate in Aspergillus (Aleksenko and Ivanova, Mol.Gen. Genet. 260 (1998) 159-164). Preferably, the replication initiating sequence is obtained from a filamentous fungal cell, more preferably a strain of *Aspergillus, Fusarium* or *Alternaria,* and even more preferably, a strain of *A*. *nidulans, A. oryzae, A. niger, F. oxysporum* or *Alternaria altenata.*

A fungal replication initiating sequence may be identified by methods well-known in the art. For instance, the sequence may be identified among genomic fragments derived from the organism in question as a sequence capable of sustaining autonomous replication in yeast, (Ballance and Turner, Gene, 36 (1985), 321-331), an indication of a capability of autonomous replication in filamentous fungal cells. The replication initiating activity in fungi of a given sequence may also be determined by transforming fungi with contemplated plasmid replicators and selecting for colonies having an irregular morphology, indicating loss of a sectorial plasmid which in turn would lead to lack of growth on selective medium when selecting for a gene found on the plasmid (Gems et al, Gene, 98 (1991) 61-67). AMA1 was isolated in this way. An alternative way to isolate a replication initiating sequence is to isolate natural occurring plasmids (eg as disclosed by Tsuge et al., Genetics 146 (1997) 111-120 for *Alternaria aternata*)*.*

Examples of fungal replication initiating sequences include, but are not limited to, the ANS1 and AMA1 sequences of *Aspergillus nidulans,* e.g., as described, respectively, by Cullen, D., et al. (1987, Nucleic Acids Res. 15:9163-9175) and Gems, D., et al. (1991, Gene 98:61-67).

Preferred embodiments relate to methods of the first aspect of the invention, wherein the fungal replication initiation sequence of step (ii) comprises the nucleic acid sequence set forth in SEQ ID NO: 1 or SEQ ID NO: 2 (for further details relating to theses sequences see WO 00/24883 SEQ ID NO: 1 and 2), or is a functional derivative thereof, preferably the functional derivative is at least 80% identical to SEQ ID NO: 1 or SEQ ID NO: 2.

The term "replication initiating activity" is used herein in its conventional meaning, i.e. to indicate that the sequence is capable of supporting autonomous replication of an extrachromosomal molecule, such as a plasmid or a DNA vector in a fungal cell.

The term "without structural rearrangement of the plasmid" is used herein to mean that no part of the plasmid is deleted or inserted into another part of the plasmid, nor is any host genomic DNA inserted into the plasmid. The replication initiating sequence to be used in the methods of the present invention is a nucleotide sequence having at least 50% identity with the nucleic acid sequence of SEQ ID NO: 1 or SEQ ID NO: 2, and is capable of initiating replication in a fungal cell; or a subsequence of (a) or (b), wherein the subsequence is capable of initiating replication in a fungal cell.

In a preferred embodiment, the nucleotide sequence has a degree of identity to the nucleic acid sequence shown in SEQ ID NO: 1 or SEQ ID NO: 2 of at least 50%, more preferably at least 60%, even more preferably at least 70%, even more preferably at least 80%, even more preferably at least 90%, and most preferably at least 97% identity (hereinafter "homologous polynucleotide"). The homologous polynucleotide also encompasses a subsequence of SEQ ID NO: 1 or SEQ ID NO: 2, which has replication initiating activity in fungal cells.

The relatedness between two amino acid sequences is described by the parameter "identity".

For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence ("invention sequence") and a different amino acid sequence ("foreign sequence") is calculated as the number of exact matches in an alignment of the two sequences, divided by the length of the "invention sequence" or the length of the "foreign sequence", whichever is the shortest. The result is expressed in percent identity.

An exact match occurs when the "invention sequence" and the "foreign sequence" have identical amino acid residues in the same positions of the overlap. The length of a sequence is the number of amino acid residues in the sequence.

For purposes of the present invention, the degree of identity between two nucleotide sequences is determined by the Wilbur-Lipman method (Wilbur and Lipman, 1983, Proceedings of the National Academy of Science USA 80: 726-730) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

The techniques used to isolate or clone a nucleic acid sequence having replication initiating activity are known in the art and include isolation from genomic DNA or cDNA. The cloning from such DNA can be effected, e.g., by using methods based on polymerase chain reaction (PCR) to detect cloned DNA fragments with shared structural features. (See, e.g., Innis, et al., 1990, PCR: A Guide to Methods and Application, Academic Press, New York.) Other nucleic acid amplification procedures such as ligase chain reaction (LCR) may be used.

In preferred embodiment, the replication initiating sequence has the nucleic acid sequence set forth in SEQ ID NO: 1 or SEQ ID NO: 2, or a respective functional subsequence thereof. For instance, a functional subsequence of SEQ ID NO: 1 is a nucleic acid sequence encompassed by SEQ ID NO: 1 or SEQ ID NO: 2 except that one or more nucleotides from the 5' and/or 3' end have been deleted. Preferably, a subsequence contains at least 100 nucleotides, more preferably at least 1000 nucleotides, and most preferably at least 2000 nucleotides. In a more preferred embodiment, a subsequence of SEQ ID NO: 1 contains at least the nucleic acid sequence shown in SEQ ID NO: 2.

### Crippled Translational Initiator Sequences

The term "translational initiator sequence" is defined herein as the ten nucleotides immediately upstream of the initiator or start codon of the open reading frame of a polypeptide-encoding nucleic acid sequence. The initiator codon encodes for the amino acid methionine, the so-called "start" codon. The initiator codon is typically an ATG, but may also be any functional start codon such as GTG. It is well known in the art that uracil (uridine), U, replaces the deoxynucleotide thymine (thymidine), T, in RNA.

In a particular embodiment according to the invention the method as described above can be further improved by using the following sequence as translation initiation start site of the marker gene comprised on the expression vector:
N YNN **ATG** YNN (SEQ ID NO: 3)
wherein "Y" in position -3 is a pyrimidin (Cytidine or Thymidine/Uridine), "N" is any nucleotide, and the numerical designations are relative to the first nucleotide in the start-codon "ATG" (in bold) of the marker;

The term "crippled translational initiator sequence" is defined herein as the ten nucleotides immediately upstream of the initiator codon of the open reading frame of a polypeptide-encoding nucleic acid sequence, wherein the initiator sequence comprises a T at the -3 position and a T at one or more of the -1, -2, and -4 positions.

Accordingly, in a preferred embodiment of the invention the sequence SEQ ID NO: 3 comprises a Thymidin (Uridin) in the -3 position; even more preferably the sequence SEQ ID NO: 3 further comprises a Thymidin (Uridin) in one more of the positions -1, -2, and -4.

The term "operably linked" is defined herein as a configuration in which a control sequence, e.g., a crippled translational initiator sequence, is appropriately placed at a position relative to a coding sequence such that the control sequence directs the production of a polypeptide encoded by the coding sequence.

The term "coding sequence" is defined herein as a nucleic acid sequence that is transcribed into mRNA which is translated into a polypeptide when placed under the control of the appropriate control sequences. The boundaries of the coding sequence are generally determined by the start codon located at the beginning of the open reading frame of the 5' end of the mRNA and a stop codon located at the 3' end of the open reading frame of the mRNA. A coding sequence can include, but is not limited to, genomic DNA, cDNA, semisynthetic, synthetic, and recombinant nucleic acid sequences.

The crippled translational sequence results in inefficient translation of the gene encoding the selectable marker. When a fungal host cell harbouring an expression vector comprising a polynucleotide encoding a polypeptide of interest physically linked with a second polynucleotide comprising a crippled translational initiator sequence operably linked to a gene encoding a selectable marker, is cultured under conditions that select for multiple copies of the selectable marker, the copy number of the polypeptide-encoding polynucleotide cloned into the vector is also increased.

The term "selectable marker" is defined herein as a gene the product of which provides for biocide or viral resistance, resistance to heavy metals, prototrophy to auxotrophs, and the like, which permits easy selection of transformed cells. Selectable markers for use in a filamentous fungal host cell include, but are not limited to, *amdS* (acetamidase), *argB* (omithine carbamoyltransferase), *bar* (phosphinothricin acetyltransferase), *hygB* (hygromycin phosphotransferase), *niaD* (nitrate reductase), *pyrG* (orotidine-5'-phosphate decarboxylase), *sC* (sulfate adenyltransferase), *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.* Functional derivatives of these selectable markers are also of interest in the present invention, in particular those functional derivatives which have decreased activity or decreased stability, thereby enabling a selection for a higher copy-number of the expression vector without increasing the concentration of the selective substance(s).

Accordingly, a preferred embodiment is a method of the first aspect, wherein the selectable marker of step (i) is selected from the group of markers consisting of *amdS, argB, bar, hygB, niaD, pyrG, sC, and trpC;* preferably the selectable marker of step (i) is *pyrG* or a functional derivative thereof, more preferably the selectable marker of step (i) is a functional derivative of *pyrG* which comprises a substitution of one or more amino acids, and most preferably the derivative comprises the amino acid substitution T102N.

The term "copy number" is defined herein as the number of molecules, per genome, of a gene which is contained in a cell. Methods for determining the copy number of a gene are will known in the art and include Southern analysis, quantitative PCR, or real time PCR.

The fungal host cell preferably contains at least two copies, more preferably at least ten copies, even more preferably at least one hundred copies, most preferably at least five hundred copies, and even most preferably at least one thousand copies of the expression cloning vector.

### Polypeptide encoding polynucleotides

The polypeptide of interest may be native or heterologous to the filamentous fungal host cell of interest. The term "heterologous polypeptide" is defined herein as a polypeptide which is not native to the fungal cell, a native polypeptide in which modifications have been made to alter the native sequence, or a native polypeptide whose expression is quantitatively altered as a result of a manipulation of the fungal cell by recombinant DNA techniques. The polynucleotide encoding the polypeptide of interest may originate from any organism capable of producing the polypeptide of interest, including multicellular organisms and microorganisms e.g. bacteria and fungi. Alternatively the polynucleotide may be a synthetically generated polynucleotide, e.g. a codon optimized polynucleotide or a shuffled polynucleotide.

A preferred embodiment of the invention relates to methods of the first aspect, wherein the organism of step (a) capable of producing one or more polypeptides of interest is a eukaryote, preferably the eukaryote is a fungus, and most preferably a filamentous fungus.

The term "polypeptide" is not meant herein to refer to a specific length of the encoded product and, therefore, encompasses peptides, oligopeptides, and proteins. Preferably, the polypeptide of interest is an enzyme, an enzyme variant, or a functional derivative thereof, more preferably the enzyme or enzyme variant is an oxidoreductase, transferase, hydrolase, lyase, isomerase, or ligase; and most preferably the enzyme or enzyme variant is an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, invertase, laccase, lipase, mannosidase, mutanase, oxidase, a pectinolytic enzyme, peroxidase, phytase, polyphenoloxidase, proteolytic enzyme, ribonuclease, transglutaminase, or xylanase.

Preferably, the polypeptide is a hormone or hormone variant or a functional derivative thereof, a receptor or receptor variant or a functional derivative thereof, an antibody or antibody variant or a functional derivative thereof, or a reporter.

In a preferred embodiment, the polypeptide is secreted extracellularly. In a more preferred embodiment, the polypeptide is an oxidoreductase, transferase, hydrolase, lyase, isomerase, or ligase. In an even more preferred embodiment, the polypeptide is an aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phospholipase, phytase, polyphenoloxidase, proteolytic enzyme, ribonuclease, transglutaminase, or xylanase.

The nucleic acid sequence encoding a polypeptide of interest may be obtained from any prokaryotic, eukaryotic, or other source. For purposes of the present invention, the term "obtained from" as used herein in connection with a given source shall mean that the polypeptide is produced by the source or by a cell in which a gene from the source has been inserted.

The techniques used to synthesize, isolate or clone a nucleic acid sequence encoding a polypeptide of interest are known in the art and include isolation from genomic DNA, preparation from cDNA, or a combination thereof. The cloning of the nucleic acid sequence from such genomic DNA can be effected, *e.g*., by using the well known polymerase chain reaction (PCR). See, for example, Innis et al., 1990, PCR Protocols: A Guide to Methods and Application, Academic Press, New York. The cloning procedures may involve excision and isolation of a desired nucleic acid fragment comprising the nucleic acid sequence encoding the polypeptide, insertion of the fragment into a vector molecule, and incorporation of the recombinant vector into the mutant fungal cell where multiple copies or clones of the nucleic acid sequence will be replicated. The nucleic acid sequence may be of genomic, cDNA, RNA, semisynthetic, synthetic origin, or any combinations thereof.

In the methods of the present invention, the polypeptide may also include a fused or hybrid polypeptide in which another polypeptide is fused at the N-terminus or the C-terminus of the polypeptide or fragment thereof. A fused polypeptide is produced by fusing a nucleic acid sequence (or a portion thereof) encoding one polypeptide to a nucleic acid sequence (or a portion thereof) encoding another polypeptide. Techniques for producing fusion polypeptides are known in the art, and include, ligating the coding sequences encoding the polypeptides so that they are in frame and expression of the fused polypeptide is under control of the same promoter(s) and terminator. The hybrid polypeptide may comprise a combination of partial or complete polypeptide sequences obtained from at least two different polypeptides wherein one or more may be heterologous to the mutant fungal cell.

Once a transformed host cell has been selected which produces the polypeptide of interest according to the methods of the invention, the encoding polynucleotide can be isolated from the selected transformed host cell, and a further optimized expression system can be designed.

Accordingly, a preferred embodiment relates to methods of the first aspect, wherein subsequently to step (d) the polynucleotide coding for the polypeptide of interest is isolated from the selected transformed host cell of step (d).

### Nucleic Acid Constructs

The present invention also relates to nucleic acid constructs comprising a polynucleotide encoding the polypeptide of interest. The polynucleotides are operably linked to one or more control sequences which direct the expression of the coding sequence in a suitable host cell under conditions compatible with the control sequences. Expression will be understood to include any step involved in the production of the polypeptide including, but not limited to, transcription, post-transcriptional modification, translation, post-translational modification, and secretion.

"Nucleic acid construct" is defined herein as a nucleic acid molecule, either single- or double-stranded, which is isolated from a naturally occurring gene, created synthetically or which has been modified to contain segments of nucleic acid combined and juxtaposed in a manner that would not otherwise exist in nature. The term nucleic acid construct is synonymous with the term expression vector when the nucleic acid construct comprises a second polynucleotide encoding a polypeptide of interest and all the control sequences required for its expression.

An isolated polynucleotide encoding a polypeptide may be further manipulated in a variety of ways to provide for expression of the polypeptide. Manipulation of the nucleic acid sequence prior to its insertion into a vector may be desirable or necessary depending on the expression vector. The techniques for modifying nucleic acid sequences utilizing recombinant DNA methods are well known in the art.

In the methods of the present invention, the nucleic acid sequences may comprise one or more native control sequences or one or more of the native control sequences may be replaced with one or more control sequences foreign to the nucleic acid sequence for improving expression of the coding sequence in a host cell.

The term "control sequences" is defined herein to include all components which are necessary or advantageous for the expression of a polypeptide of interest. Each control sequence may be native or foreign to the nucleic acid sequence encoding the polypeptide. Such control sequences include, but are not limited to, a leader, polyadenylation sequence, propeptide sequence, crippled translational initiator sequence of the present invention, signal peptide sequence, and transcription terminator. At a minimum, the control sequences include translational initiator sequences, and transcriptional and translational stop signals. The control sequences may be provided with linkers for the purpose of introducing specific restriction sites or cloning sites facilitating ligation of the control sequences with the coding region of the nucleic acid sequence encoding a polypeptide.

The control sequence may be an appropriate promoter sequence, a nucleic acid sequence which is recognized by a host cell for expression of the nucleic acid sequence. The promoter sequence contains transcriptional control sequences which mediate the expression of the polypeptide. The promoter may be any nucleic acid sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*)*, Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase, *Fusarium oxysporum* trypsin-like protease (WO 96/00787), as well as the NA2-tpi promoter (a hybrid of the promoters from the genes for *Aspergillus niger* neutral alpha-amylase and *Aspergillus oryzae* triose phosphate isomerase); and mutant, truncated, and hybrid promoters thereof.

In a particular embodiment of the first aspect, the promoter of step (iii) is the promoter from the neutral amylase encoding gene (NA2) from *Aspergillus niger* disclosed in WO 89/01969. In another particular embodiment the promoter is the NA2-tpi promoter (a hybrid of the promoter from the genes encoding *Aspergillus niger* neutral alpha-amylase and the untranslated leader from the *Aspergillus oryzae* triose phosphate isomerase (tpi) promoter).

The control sequence may be a suitable transcription terminator sequence, a sequence recognized by a host cell to terminate transcription. The terminator sequence is operably linked to the 3' terminus of the nucleic acid sequence encoding the polypeptide. Any terminator which is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

A preferred embodiment relates to methods of the first aspect, wherein the transcription terminator of step (iii) is the terminator from the glucoamylase encoding gene (AMG) from *Aspergillus niger* (Boel,E.; Hjort, I.; Svensson,B.; Norris,F.; Norris,K.E.; Fiil,N.P., Glu-coamylases G1 and G2 from Aspergillus niger are synthesized from two different but closely related mRNAs. EMBO J. 3:1097 (1984)).

The control sequence may also be a suitable leader sequence, a nontranslated region of an mRNA which is important for translation by the host cell. The leader sequence is operably linked to the 5' terminus of the nucleic acid sequence encoding the polypeptide. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

A preferred embodiment relates to methods of the first aspect, wherein the promoter is operably linked, upstream of the cloning-site of step (iii), to the polynucleotide encoding the leader peptide of triose phosphate isomerase (tpiA) from *Aspergillus nidulans.* (Mcknight G.L., O'Hara P.J., Parker M.L.,"Nucleotide sequence of the triosephosphate isomerase gene from Aspergillus nidulans: Implications for a differential loss of introns",Cell 46:143-147(1986)).

The control sequence may also be a polyadenylation sequence, a sequence operably linked to the 3' terminus of the nucleic acid sequence and which, when transcribed, is recognized by the host cell as a signal to add polyadenosine residues to transcribed mRNA. Any polyadenylation sequence which is functional in the host cell of choice may be used in the present invention.

Preferred polyadenylation sequences for filamentous fungal host cells are obtained from the genes for *Aspergillus oryzae* TAKA amylase, *Aspergillus niger* glucoamylase, As*pergillus nidulans* anthranilate synthase, *Fusarium oxysporum* trypsin-like protease, and As*pergillus niger* alpha-glucosidase.

The control sequence may also be a signal peptide coding region that codes for an amino acid sequence linked to the amino terminus of a polypeptide and directs the encoded polypeptide into the cell's secretory pathway. The 5' end of the coding sequence of the nucleic acid sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for filamentous fungal host cells are the signal peptide coding regions obtained from the genes for *Aspergillus oryzae* TAKA amylase, As*pergillus niger* neutral amylase, *Aspergillus niger* glucoamylase, *Rhizomucor miehei* aspartic proteinase, *Humicola insolens* cellulase, and *Humicola lanuginosa* lipase.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (aprE), *Bacillus subtilis* neutral protease (*nprT*)*, Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

### Expression Vectors

The various nucleic acid and control sequences described above may be joined together to produce a recombinant expression vector which may include one or more convenient restriction sites to allow for insertion or substitution of the promoter and/or nucleic acid sequence encoding the polypeptide at such sites. Alternatively, the nucleic acid sequence may be expressed by inserting the nucleic acid sequence or a nucleic acid construct comprising the crippled translational initiator sequence and/or sequence into an appropriate vector for expression. In creating the expression vector, the coding sequence is located in the vector so that the coding sequence is operably linked with a crippled translational initiator sequence of the present invention and one or more appropriate control sequences for expression.

The recombinant expression vector may be any vector (*e.g*., a plasmid or virus) which can be conveniently subjected to recombinant DNA procedures and can bring about the expression of a nucleic acid sequence. The choice of the vector will typically depend on the compatibility of the vector with the host cell into which the vector is to be introduced. The vectors may be linear or closed circular plasmids.

The vector may be an autonomously replicating vector, *i.e*., a vector which exists as an extrachromosomal entity, the replication of which is independent of chromosomal replication, *e.g*., a plasmid, an extrachromosomal element, a minichromosome, or an artificial chromosome. The vector may contain any means for assuring self-replication.

The vectors of the present invention also contain one or more selectable markers which permit easy selection of transformed cells as described earlier.

For autonomous replication, the vector further comprises an origin of replication enabling the vector to replicate autonomously in the host cell in question. In a particular embodiment the ARS is an AMA-1 sequence as described above.

The procedures used to ligate the elements described above to construct the recombinant expression vectors of the present invention are well known to one skilled in the art (see, e.g., Sambrook *et al.,* 1989, *supra*)*.*

### Host Cells

The host cell may be any fungal cell useful in the methods of the present invention. "Fungi" as used herein includes the phyla Ascomycota, Basidiomycota, Chytridiomycota, and Zygomycota (as defined by Hawksworth et al., In, Ainsvvorth and Bisby's Dictionary of The Fungi, 8th edition, 1995, CAB International, University Press, Cambridge, UK) as well as the Oomycota (as cited in Hawksworth *et al.,* 1995, *supra,* page 171) and all mitosporic fungi (Hawksworth *et al.,* 1995, *supra*)*.*

In a preferred embodiment, the fungal host cell is a filamentous fungal cell. "Filamentous fungi" include all filamentous forms of the subdivision Eumycota and Oomycota (as defined by Hawksworth *et al.,* 1995, *supra*)*.* The filamentous fungi are characterized by a my-celial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

In a preferred embodiment, the filamentous fungal host cell is a cell of a species of, but not limited to, *Acremonium, Aspergillus, Coprinus, Fusarium, Humicola, Mucor, Myceliophthora, Neurospora, Penicillium, Thielavia, Tolypocladium,* or *Trichoderma.*

In a more preferred embodiment, the filamentous fungal host cell is an *Aspergillus awamori, Aspergillus foetidus, Aspergillus japonicus, Aspergillus nidulans, Aspergillus niger* or *Aspergillus oryzae* cell. In a particular embodiment the *Aspergillus* host cell is *Aspergillus oryzae* or *Aspergillus niger.* In another most preferred embodiment, the filamentous fungal host cell is a *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell. In a particular embodiment the *Fusarium* host cell is *Fusarium oxysporum.* In another most preferred embodiment, the filamentous fungal host cell is a *Humicola insolens, Humicola lanuginosa, Mucor miehei, Myceliophthora thermophila, Neurospora crassa, Penicillium purpurogenum, Thielavia terrestris, Trichoderma harzianum, Trichoderma koningii, Trichoderma longibrachiatum, Trichoderma reesei,* or *Trichoderma viride* cell. In a particular embodiment the host cell is *Trichoderma reesei.*

In an even most preferred embodiment, the *Fusarium venenatum* cell is *Fusarium venenatum* A3/5, which was originally deposited as *Fusarium graminearum* ATCC 20334 and recently reclassified as *Fusarium venenatum* by Yoder and Christianson, 1998, Fungal Genetics and Biology 23: 62-80 and O'Donnell et al., 1998, Fungal Genetics and Biology 23: 57-67; as well as taxonomic equivalents of *Fusarium venenatum* regardless of the species name by which they are currently known. In another preferred embodiment, the *Fusarium venenatum* cell is a morphological mutant of *Fusarium venenatum* A3/5 or *Fusarium venenatum* ATCC 20334, as disclosed in WO 97/26330.

Fungal cells may be transformed by a process involving protoplast formation, transformation of the protoplasts, and regeneration of the cell wall in a manner known *per se.* Suitable procedures for transformation of *Aspergillus* host cells are described in EP 238 023 and Yelton et al., 1984, Proceedings of the National Academy of Sciences USA 81: 1470-1474. Suitable methods for transforming *Fusarium* species are described by Malardier et al., 1989, Gene 78: 147-156 and WO 96/00787.

The present invention is further described by the following examples.

### EXAMPLES

### Materials and Methods

### Strains:

*Aspergillus oryzae* NBRC4177: available from Institute for fermentation, Osaka; 17-25 Juso Hammachi 2-Chome Yodogawa-Ku, Osaka, Japan.

*Aspergillus oryzae* Jal355 (amy⁻, alp⁻, Npl⁻, CPA⁻, KA⁻, pyrG) is a derivative of *A. oryzae* A1560 wherein the *pyrG* gene has been inactivated, as described in WO 98/01470; transformation protocol as described in WO 00/24883.

*Aspergillus oryzae* PFjo218 (amy⁻, alp⁻, Npl⁻, CPA⁻, KA⁻, pyrG⁻, ku70⁻) is described in Example 1.

*Aspergillus oryzae* PFjo220 (amy⁻, alp⁻, Npl⁻, CPA⁻, KA⁻, pyrG⁻, ku70::PyrG) is described in Example 1.

### Plasmids:

pENI2344 is described in WO2003/070956.
pENI2155 is described in WO2003/070956.
pIC19H is described in Marsh et al., 1984, Gene 32:481-485.
pJaL554 is described in patent WO2001068864, example 8.
pJaL925 is described in example 4.
pJaL575 is described in example 1.
pDV8 is described in patent WO 0168864, example 8

### Genes

pyrG: This gene codes for orotidine-5'-phosphate decarboxylase, an enzyme involved in the biosynthesis of uridine.
wA: This gene codes for a polyketide synthase, an enzyme involved in the spore (conidia) colour formation. Disruption of the wA gene gives a white spore colour.

### Transformation Aspergillus oryzae JaL355 and PFjo218

A 16-20 hour old culture was harvested in Mira cloth and washed with 0,6 M MgSO₄. The mycelia was transferred to a 100 ml plastic tube containing 10 ml MgSO₄, 10 mM NaH₂PO₄, pH 5.8, 50-100 mg Glucanex. Twelve mg BSA was added and the solution was incubated for 2 hours at 37°C under weak shaking. Protoplasts were havested through Mira cloth and 5 ml ST (0,6 M sorbitol, 100 mM Tris-Cl, pH 7,0) was added as a layer upon the protoplasts. After 15 minutes centrifugation at 2500 rpm the protoplast in the interfase was harvested and transferred to a new tube. Two volume of STC (1,2 M sorbitol, 10 mM Tris-Cl, pH 7,5, 10 Mm CaCl₂) was added followed by 5 minutes centrifugation at 2500 rpm. The protoplasts were washed twice in 5 ml STC and finally resuspended in STC.

Approx. 1-4 ug DNA was added to 100 µl of protoplasts followed by addition of 300 ul 60 % PEG 4000. The protoplasts were incubated for 20 minutes at room temperature followed by dilution in 1.2 ml sorbitol and centrifugation for 10 minutes at 2500 rpm. The transformants were resuspended in 100 µl sorbitol and plated on selective medium (WO 00/24883).

### PCR amplification

All PCR amplifications were performed in volumes of 50 microL. containing 1.75 units of Expand High Fidelity PCR System (Roche), approx. 1 micro g DNA, 250 microM of each dNTP, and 10 pmol of each of the two primers described above in Expand High Fidelitybuffer with 1,5 mM MgCl₂.

Amplification was carried out in a MJ Research PCT-220 DNA Engine Dyad^{™} Peltier Thermal Cycler, and consisted of cycle of 2 minutes at 94°C, followed by 25 cycles of 30 seconds at 94°C, 1 min at 55°C, and 1.5 minutes at 72°C, followed by an extra extension of 5 minutes at 72°C.

### Lipase Assay:

Transformants to be assayed for lipase activity were inoculated in a 96 well microtiter plate containing 1*Vogel medium and 2 % maltose (Methods in Enzymology, vol. 17, p. 84). After 4 days growth at 34°C, the culture broth was assayed for lipase activity using pnp-valerate as a lipase substrate.

A 10 microliter aliquot of media from each well was added to a microtiter well containing 200 microliter of a lipase substrate of 0.018% p-nitrophenylvalerate, 0.1% Triton X™-100, 10 mM CaCl₂, 50 mM Tris pH 7.5. Lipase activity was assayed spectrophotometrically at 15-second intervals over a five minute period, using a kinetic microplate reader (Molecular Device Corp., Sunnyvale CA), using a standard enzymology protocol (e.g., Enzyme Kinetics, Paul C.Engel, ed., 1981, Chapman and Hall Ltd.). Briefly, product formation is measured during the initial rate of substrate turnover and is defined as the slope of the curve calculated from the absorbance at 405 nm every 15 seconds for 5 minutes. For each group of transformants an average value and the relative standard deviations were calculated.

### Genomic DNA preparation:

Fungal mycelia (harvested after growth in 10 ml YPD for 2 days) is dried in a speedy vac and the mycelium is crushed. 1 ml of lysis buffer is added (Lysis buffer: 100 mM EDTA, 10 mM Tris-pH 8.0, 1 % Triton X 100, 500 mM Guanidinium-HCl, 200 mM NaCl), mixed, 2 microliter (10 mg/ml) RNAseA is added, incubated for 30 mins at 37°C. 5 microliter proteinase K (20 mg/ml) is added. Mix and incubate for 2 hours at 50°C. Spin 10 mins at 20.000g. Load the supernatant to a plasmid spin cup (from QIAprep®Miniprep kit, Qiagen) and follow the kit protocol. Elute the genomic DNA in 100 microliter.

### Example 1

### Construction of a KU70 deleted Aspergillus orvzae strain

### Primers:

| Primer | |
|---|---|
| #313/KU-5'-rev-deltakons. | TGATTACGCCAAGCTTGCGGCCGCGACCAAAGCGTGCGAATAGCG (SEQ ID NO: 4) |
| #314/KU-5'-forw-deltakons | TGCAGCTGATAAGCTTGGTGACTATAAACAATCGCCG (SEQ ID NO: 5) |
| #315/KU-3'-forw-deltakons | GACGGCCAGTGAATTCGCGGCCGCCGAGGAACTCTGCTACTGCC (SEQ ID NO: 6) |
| #316/KU-3'-rev-deltakons | TACCGAGCTCGAATTCGGGCCGACGAGTTGGAAAAGC (SEQ ID NO: 7) |
| #340/A.flav KU70 rev2 | GGAGTCCGTATAGTAAGCATC (SEQ ID NO: 8) |
| #105/niaDp-forw #1 | TGTACAGCACTGTATTGGTATGTGAACG (SEQ ID NO: 9) |
| #126/pyrG-tjek-rev1 | CATGTAGATAAGATTAGGGC (SEQ ID NO: 10) |
| #172450 | |
| #172449 | GACGAATTCCGATGAATGTGTGTCCTG (SEQ ID NO: 12) |

The single restriction endonuclease sites BamHI and BgIII in pDV8 were removed by two succeeding rounds of cutting with each of the restriction endonucleases and the free overhang-ends were filled out by treatment with Klenow polymerase and the four deoxyribonucleotides and ligated resulting in plasmid pJaL504.

From pJaL504 a 2514 bp fragment were amplified by PCR with primer 172450 and 172449 (SEQ ID NO: 11 and 12) and cloned into the vector pCR^{®}4Blunt-TOPO resulting in plasmid pJaL575.

pPFJo118 consists of pUC19 (GenBank/EMBL accession number L09137) in which a 1185 bp HindIII-Asp718 fragment, containing the A. oryzae pyrG gene flanked by an extra copy of the promoter, was cloned into the same sites (see sequence of pyrG+repeat below). 1 kb of the KU70 3' flanking region was PCR amplified with primers #315 and #316 and cloned via BD In-Fusion™ Cloning Kit (BD Biosciences Clontech) into pPFJo118, *Eco*RI opened - resulting in pPFJo209. 1 kb of the KU70 5' flanking region was PCR amplified with primers #313 and #314 inserted into the pCR®4Blunt-TOPO® vector (using the Zero Blunt® TOPO® PCR Cloning Kit for Sequencing from Invitrogen). From here the KU70 5'-end was cut out with EcoRI, bluntended using the Klenow fragment (30 mins at room temperature), and finally cloned into pPFJo209, *Hind*III opened and blunt ended as just described for the KU70 5'-end fragment. This resulted in the pPFJo210 plasmid. pPFJo210 was opened with Ndel, blunt ended and ligated to the *Eco*RI-*Eco*RI fragment from pJaL575, containing the HSV-tk counter selection cassette, also bluntended - this resulted in the final KU70 deletion construct pPFJo247.

Approximately 40 portions (40 x 100 microliter) JaL355 were transformed with pPFJo247 linearized with *Bst*XI*.* Transformants were selected on Sucrose medium containing the nucleoside analogue 5-fluoro-2'-deoxyuridine (FDU), which allows for de-selection of transformants in which only a single crossover has occurred - since expression from the HSV-tk cassette is fatal for the cell in the presence of FDU. 19 transformants appeared after transformation and they were all re-isolated onto new Sucrose+FDU plates after 10 day of incubation at 37°C. Genomic DNA was isolated from these transformants and a preliminary PCR screen was performed, using one primer in the genomic region outside the flanks used for the deletion (#340) and one in the pyrG marker in between the flanks (#126). From this PCR screen, 10 transformants gave the right PCR band of 1114 bp, and these 10 transformants were controlled by Southern blot analysis. For Southern blots genomic DNA was prepared as described in Materials and Methods. Genomic DNA was digested with Xhol-Pvul and XhoI-BgIII resulting in bands on the Southern blot hybridizing to bands of 5417 bp and 3448 bp respectively for the correctly KU70 deletion strain, when using KU70 3'-end as probe (PCR amplified using primers # 315 and 316 resulting in a product of 1002 bp). Wild type genomic DNA gave bands of 4659 bp and 2142 bp respectively. All 10 transformants tested proved to be correctly disrupted. Transformant #JaL355/pPFJo247-19 was selected and named PFJo220. This transformant was streaked on slants and from there it was washed of using spore solution and a thick spore suspension was plated onto 5-FOA-plates (containing 5-fluoro orotic acid) in order to select for strains in which the pyrG gene had looped out - leaving the strain ku70 deleted and pyrG⁻. One such strain was isolated and named PFJo218.

### Example 2

### Expression of lipase in JaL355 and PFjo218

The *Aspergillus* strains Jal355 and Pfjo218 were both transformed with pENI2344 as described. pENI2344 is described in detail in WO2003/070956 (Example 2) and contains a lipase gene as a reporter gene and pyrG as a selection marker. The pyrG gene on pENI2344 comprises the point mutation,T102N, resulting in an increase in copy number.

11 transformants from each strain were inoculated in 200 µl media (1*vogel, 2 %maltose). After 4 days growth at 34 degrees C the inoculums was transferred to plates and grown for 3 days at 37 degrees, the 11 transformants of each strain were inoculated in 200 µl Yeast peptone + 2% maltose and grown for 4 days at 34 degrees C.
10 µl media was assayed using the lipase substrate pnp-valerate as described above and in WO2003070956. The result showed that there was a relative standard deviation of 34 % between the lipase expression levels of the 11 independent JaL355 *Aspergillus* transformants. The relative standard deviation for the expression levels of lipase in the 11 independent PFjo218 transformants was only 18%. This shows that deletion/inactivation of the ku70 gene leads to more uniform expression levels, which is desirable when screening gene variant libraries.

### Example 3

### Expression of lipase in JaL355 and PFjo218

The *Aspergillus oryzae* strains JaL355 and PFjo218 were both transformed with the plasmid pENI2155 comprising a lipase reporter gene.

Plasmid pENI2155 comprises a crippled kozak region upstream of the *pyrG* gene, and is constructed as described in WO 2003/070956 (Example 1). The plasmid is basically identical to pENI2344 except that the *pyrG* gene is wild type. Details on the construction can be found in WO 2003/070956 (Example 1).

About 10 transformants from each strain were inoculated in 200 µl media (2 %maltose + YP). After 4 days of growth at 34 degrees C, the culture media was assayed for lipase activity using pnp-valerate. The inoculums was transferred to plates and grown for 3 days at 37 degrees C.

The lipase assay was repeated a second time after a total of 14 days of growth. About 10 transformants of each strain was inoculated in 200 µl Yeast peptone + 2% maltose and grown for 3 days at 34 degrees Celsius. After 4 days growth at 34 degrees C the culture media was assayed for lipase activity using pnp-valerate (WO2003070956). The inoculums were transferred to plates and grown for 3 days at 37 degrees.

Finally the lipase assay was repeated a third time after a total of 21 days of growth. About 10 transformants of each strain was inoculated in 200 µl Yeast peptone + 2% maltose and grown for 4 days at 34 degrees Celsius. After 4 days growth at 34 degrees the culture media was assayed for lipase activity using pnp-valerate (WO2003070956).

### Result:

The expression data from the lipase assays are shown in the table below. It is evident that there is a low relative standard deviation, when using the ku70 deleted strain. This low relative standard deviation in expression level is desirable, when screening a variant library.

| Days after transformation | JAL355 Relative st. dev | PFjo218 Relative st. dev |
|---|---|---|
| 7 | 24% | 12% |
| 13 | 45% | 15% |
| 20 | 45% | 18% |

### Example 4

### Determination of NHR/HR frequency in KU70 deleted strain

In order to test the behaviour of the KU70 strain background regarding homologous recombination, different easily selectable targets can be chosen as test cases. Targets could be: wA - which result in snow white spores when disrupted, adeB - results in red mycelium when disrupted, and niaD - results in inability to grow on plates with nitrate as sole nitrogen source, whereas all strains will grow on nitrite as sole nitrogen source. The results for wA are shown in the table below.

### A. Construction of the Aspergillus oryzae wA deletion plasmid pJaL925

Plasmid pJaL901 contains a 4658 bp BgIII fragment from A.oryzae NBRC4177 encoding the wA gene (SEQ ID NO: 13) in pIC19H. Plasmid pJaL901 was digested with Asp718 and SnaBI and the 5501 bp fragment was purified. The repeat flanked pyrG selection marker from pJaL554 was purified as a 2027 bp Asp718-Smal fragment. The two fragments were ligated resulting in plasmid pJaL925. The pyrG gene thereby replaces a 1861 bp Asp718-SnaBI encoding part of the wA gene and the pyrG gene is then flanked by a 685 bp fragment of the 5' end of wA and a 2105 bp fragment of the 3' end of wA.

### B. Transformation of JaL355 and PFJo218 with pJaL925

JaL355 and PFJo218 were transformed with pJaL925 as described earlier. The results as shown in the table below clearly shows the decrease in NHR in the KU70 deleted strain.

| Transformants in: | pJaL925-wA (white/green spores) | HRF |
|---|---|---|
| JaL355 (pyrG-) | 10/500 | ~2 % |
| PFJo218 (ku70Δ, pyrG⁻) | 80/20 | ~80 % |

## Claims

1. A method for producing a recombinant polypeptide of interest, the method comprising the steps of:
a) providing a polynucleotide library encoding one or more polypeptides of interest, wherein the library was prepared in an expression cloning vector comprising at least the following elements:
i) a polynucleotide encoding a selectable marker;
ii) a fungal replication initiation sequence, preferably an autonomously replicating sequence (ARS); and
iii) a polynucleotide comprising in sequential order: a promoter derived from a fungal cell, a cloning-site into which the library is cloned, and a transcription terminator;
b) transforming a mutant of a parent filamentous fungal host cell with the library, wherein the frequency of non-homologous recombination in the mutant has been decreased compared to the parent;
c) culturing the transformed host cell obtained in (b) under conditions suitable for expression of the polynucleotide library;
d) selecting a transformed host cell which produces the polypeptide of interest; and
e) recovering the polypeptide of interest.

2. The method according to claim 1, wherein the mutant filamentous fungal host cell is modified in a locus selected from the group consisting of ku70, ku80, rad50, mre11, xrs2, lig4, sir4 or functional equivalents thereof.

3. The method according to claim 2, wherein the locus is ku70 or ku80 or functional equivalents thereof.

4. The method according to claim 1, wherein the ARS is an AMA1-sequence or a functional derivative thereof.

5. The method according to any of claims 1-4, wherein the selectable marker of step (i) is selected from the group of markers consisting of *amdS, argB, bar, hygB, niaD, pyrG, sC, and trpC.*

6. The method according to claim 5, wherein the selectable marker of step (i) is *pyrG* or a functional derivative thereof.

7. The method according to claim 6, wherein the selectable marker of step (i) is a functional derivative of *pyrG* which comprises a substitution of one or more amino acids, preferably the derivative comprises the amino acid substitution T102N.

8. The method according to any of claims 1-7, wherein the fungal replication initiation sequence of step (ii) comprises the nucleic acid sequence set forth in SEQ ID NO:1 or SEQ ID NO:2, or is a functional derivative thereof, preferably the functional derivative is at least 80% identical to SEQ ID NO:1 or SEQ ID NO: 2.

9. The method according to any of claims 1 - 8, wherein the promoter of step (iii) is the promoter from the neutral amylase encoding gene (NA2) from *Aspergillus niger.*

10. The method according to any of the claims 1 - 8, wherein the promoter is the NA2tpi promoter.

11. The method according to claim 1, wherein the transcription terminator of step (iii) is the terminator from the glucoamylase encoding gene (AMG) from *Aspergillus niger.*

12. The method according to any of claims 1 - 11, wherein the filamentous fungal host cell is of the genus *Acremonium, Aspergillus, Coprinus, Fusarium, Humicola, Mucor, Myceliopthora, Neurospora, Penicillium, Thielavia, Tolypocladium* or *Trichoderma.*

13. The method according to claim 12, wherein the cell is of the species *Aspergillus oryzae, Aspergillus niger, Aspergillus nidulans, Coprinus cinereus, Fusarium oxysporum,* or *Trichoderma reesei.*

14. The method according to any of claims 1 - 13, wherein the polypeptide of interest is an enzyme, an enzyme variant, or a functional derivative thereof.

15. The method according to claim 14, wherein the enzyme or enzyme variant is an oxidoreductase, transferase, hydrolase, lyase, isomerase, or ligase.

## Patentansprüche

1. Verfahren zum Herstellen eines rekombinanten Polypeptids von Interesse, wobei das Verfahren die Schritte umfasst:
(a) Bereitstellen einer Polynukleotidbibliothek, die ein oder mehrere Polypeptide von Interesse kodiert, wobei die Bibliothek in einem Expressionsklonierungsvektor erzeugt wurde, umfassend mindestens die folgenden Elemente:
i. ein einen Selektionsmarker kodierendes Polynukleotid;
ii. eine Replikationsinitiationssequenz aus einem Pilz, vorzugsweise eine autonom replizierende Sequenz (ARS); und
iii. ein Polynukleotid, umfassend in sequentieller Reihenfolge: einen aus einer Pilzzelle abgeleiteten Promotor, eine Klonierungsstelle, in die die Bibliothek kloniert wird, und einen Transkriptionsterminator;
(b) Transformieren einer Mutante einer Ausgangswirtszelle eines filamentösen Pilzes mit der Bibliothek, wobei die Frequenz einer nicht-homologen Rekombination in der Mutante im Vergleich zu der Ausgangszelle verringert worden ist;
(c) Kultivieren der in (b) erhaltenen transformierten Wirtszelle unter Bedingungen, die zur Expression der Polynukleotidbibliothek förderlich sind;
(d) Auswählen einer transformierten Wirtszelle, die das Polypeptid von Interesse herstellt; und
(e) Gewinnen des Polypeptids von Interesse.

2. Verfahren nach Anspruch 1, wobei die mutierte Zelle eines filamentösen Pilzes in einem Lokus modifiziert ist, ausgewählt aus der Gruppe bestehend aus ku70, ku80, rad50, mre11, xrs2, lig4, sir4 oder funktionellen Äquivalenten davon.

3. Verfahren nach Anspruch 2, wobei der Lokus ku70 oder ku80 oder funktionelle Äquivalente davon ist.

4. Verfahren nach Anspruch 1, wobei die ARS eine AMA-1-Sequenz oder ein funktionelles Derivat davon ist.

5. Verfahren nach einem beliebigen der Ansprüche 1-4, wobei der Selektionsmarker von Schritt (i) ausgewählt ist aus der Gruppe von Markern, bestehend aus *amdS, argB, bar, hygB, niaD, pyrG, sC* und *trpC.*

6. Verfahren nach Anspruch 5, wobei der Selektionsmarker von Schritt (i) *pyrG* oder ein funktionelles Derivat davon ist.

7. Verfahren nach Anspruch 6, wobei der Selektionsmarker aus Schritt (i) ein funktionelles Derivat von pyrG ist, das eine Substitution von einer oder mehreren Aminosäuren umfasst, vorzugsweise umfasst das Derivat die Aminosäuresubstitution T102N.

8. Verfahren nach einem beliebigen der Ansprüche 1-7, wobei die Replikationsinitiationssequenz aus einem Pilz aus Schritt (ii) die in SEQ ID NO: 1 oder SEQ ID NO: 2 dargestellte Nukleinsäuresequenz umfasst, oder ein funktionelles Derivat davon ist, vorzugsweise ist das funktionelle Derivat mindestens 80% identisch zu SEQ ID NO: 1 oder SEQ ID NO: 2.

9. Verfahren nach einem beliebigen der Ansprüche 1-8, wobei der Promotor von Schritt (iii) der Promotor aus dem neutrale Amylase kodierenden Gen (NA2) aus *Aspergillus niger* ist.

10. Verfahren nach einem beliebigen der Ansprüche 1-8, wobei der Promotor der NA2tpi-Promotor ist.

11. Verfahren nach Anspruch 1, wobei der Transkriptionsterminator aus Schritt (iii) der Terminator aus dem Glucoamylase kodierenden Gen (AMG) aus *Aspergillus niger* ist.

12. Verfahren nach einem beliebigen der Ansprüche 1-11, wobei die Wirtszelle eines filamentösen Pilzes von der Gattung *Acremonium, Aspergillus, Coprinus, Fusarium, Humicola, Mucor, Myceliopthora, Neurospora, Penicillium, Thielavia, Tolypocladium* oder *Trichoderma* ist.

13. Verfahren nach Anspruch 12, wobei die Zelle von der Spezies *Aspergillus oryzae, Aspergillus niger, Aspergillus nidulans, Coprinus cinereus, Fusarium oxysporum* oder *Trichoderma reesei* ist.

14. Verfahren nach einem beliebigen der Ansprüche 1-13, wobei das Polypeptid von Interesse ein Enzym, eine Enzymvariante oder ein funktionelles Derivat davon ist.

15. Verfahren nach Anspruch 14, wobei das Enzym oder die Enzymvariante eine Oxidoreduktase, Transferase, Hydrolase, Lyase, Isomerase oder Ligase ist.

## Revendications

1. Procédé de production d'un polypeptide recombinant d'intérêt, procédé comprenant les étapes de :
a) fourniture d'une bibliothèque de polynucléotides codant pour un ou plusieurs polypeptides d'intérêt, la bibliothèque ayant été préparée dans un vecteur de clonage d'expression comprenant au moins les éléments suivants :
i) un polynucléotide codant pour un marqueur sélectionnable ;
ii) une séquence fongique d'initiation de la réplication, préférablement une séquence de réplication autonome (SRA) ; et
iii) un polynucléotide comprenant dans l'ordre séquentiel: un promoteur dérivé d'une cellule fongique, un site de clonage dans lequel la bibliothèque est clonée, et un terminateur de transcription ;
b) transformation d'un mutant d'une cellule hôte fongique filamenteuse parente avec la bibliothèque, la fréquence de recombinaison non homologue dans le mutant ayant été diminuée par rapport au parent ;
c) culture de la cellule hôte transformée obtenue dans (b) dans des conditions appropriées pour l'expression de la bibliothèque de polynucléotides ;
d) sélection de la cellule hôte transformée qui produit le polypeptide d'intérêt ; et
e) récupération du polypeptide d'intérêt.

2. Procédé selon la revendication 1, dans lequel la cellule hôte fongique filamenteuse mutante est modifiée en un locus sélectionné à partir du groupe consistant en ku70, ku80, rad50, mre11, xrs2, lig4, sir4 ou leurs équivalents fonctionnels.

3. Procédé selon la revendication 2, dans lequel le locus est ku70 ou ku80 ou leurs équivalents fonctionnels.

4. Procédé selon la revendication 1, dans lequel la SRA est une séquence AMA1 ou un dérivé fonctionnel de celle ci.

5. Procédé selon l'une quelconque des revendications 1 à 4, dans lequel le marqueur sélectionnable de l'étape (i) est sélectionné à partir du groupe de marqueurs consistant en amdS, argB, bar, hygB, niaD, pyrG, sC, et trpC.

6. Procédé selon la revendication 5, dans lequel le marqueur sélectionnable de l'étape (i) est pyrG ou un dérivé fonctionnel de celui ci.

7. Procédé selon la revendication 6, dans lequel le marqueur sélectionnable de l'étape (i) est un dérivé fonctionnel de pyrG qui comprend une substitution de un ou plusieurs acides aminés, préférablement le dérivé comprenant la substitution d'acides aminés T102N.

8. Procédé selon l'une quelconque des revendications 1 à 7, dans lequel la séquence d'initiation de réplication fongique de l'étape (ii) comprend la séquence d'acides nucléiques déterminée dans SEQ ID NO:1 ou SEQ ID NO:2, ou est un dérivé fonctionnel de celle ci, préférentiellement le dérivé fonctionnel étant au moins 80% identique à SEQ ID NO :1 ou SEQ ID NO :2.

9. Procédé selon l'une quelconque des revendications 1 à 8, dans lequel le promoteur de l'étape (iii) est le promoteur du gène codant pour l'amylase neutre (NA2) d*'Aspergillus niger.*

10. Procédé selon l'une quelconque des revendications 1 à 8, dans lequel le promoteur est le promoteur NA2tpi.

11. Procédé selon la revendication 1, dans lequel le terminateur de transcription de l'étape (iii) est le terminateur du gène codant pour la glucoamylase (AMG) *d'Aspergillus niger.*

12. Procédé selon l'une quelconque des revendications 1 à 11, dans lequel la cellule hôte fongique filamenteuse est du genre *Acremonium, Aspergillus, Coprinus, Fusarium, Humicola, Mucor, Myceliopthora, Neurospora, Penicillium, Thielavia, Tolypocladium ou Trichoderma.*

13. Procédé selon la revendication 12, dans lequel la cellule est de l'espèce *Aspergillus oryzae, Aspergillus niger, Aspergillus nidulans, Coprinus cinereus, Fusarium oxysporum,* ou *Trichoderma reesei.*

14. Procédé selon l'une quelconque des revendications 1 à 13, dans lequel le polypeptide d'intérêt est une enzyme, un variant enzymatique, ou un dérivé fonctionnel de ceux ci.

15. Procédé selon la revendication 14, dans lequel l'enzyme ou le variant enzymatique est une oxydoréductase, une transférase, une hydrolase, une lyase, une isomérase, ou une ligase.


### SEQUENCE LISTING

<110> Novozymes A/S
   Vind, Jesper
<120> Expression cloning method suitable for selecting library clones producing a polypeptide of interest.
<130> 11121.204-WO
<160> 17
<170> PatentIn version 3.5
<210> 1
   <211> 5259
   <212> DNA
   <213> Aspergillus nidulans
<400> 1
   aagcttattt tttgtatact gttttgtgat agcacgaagt ttttccacgg tatcttgtaa      60
   aaatatatat ttgtggcggg cttacctaca tcaaattaat aagagactaa ttataaacta     120
   aacacacaag caagctactt tagggtaaaa gtttataaat gcttttgacg tataaacgtt     180
   gcttgtattt attattacaa ttaaaggtgg atagaaaacc tagagactag ttagaaacta     240
   atctcaggtt tgcgttaaac taaatcagag cccgagaggt taacagaacc tagaagggga     300
   ctagatatcc gggtagggaa acaaaaaaaa aaaacaagac agccacatat tagggagact     360
   agttagaagc tagttccagg actaggaaaa taaaagacaa tgataccaca gtctagttga     420
   caactagata gattctagat tgaggccaaa gtctctgaga tccaggttag ttgcaactaa     480
   tactagttag tatctagtct cctataactc tgaagctaga ataacttact actattatcc     540
   tcaccactgt tcagctgcgc aaacggagtg attgcaaggt gttcagagac tagttattga     600
   ctagtcagtg actagcaata actaacaagg tattaaccta ccatgtctgc catcaccctg     660
   cacttcctcg ggctcagcag ccttttcctc ctcattttca tgctcatttt ccttgtttaa     720
   gactgtgact agtcaaagac tagtccagaa ccacaaagga gaaatgtctt accactttct     780
   tcattgcttg tctcttttgc attatccatg tctgcaacta gttagagtct agttagtgac     840
   tagtccgacg aggacttgct tgtctccgga ttgttggagg aactctccag ggcctcaaga     900
   tccacaacag agccttctag aagactggtc aataactagt tggtctttgt ctgagtctga     960
   cttacgaggt tgcatactcg ctccctttgc ctcgtcaatc gatgagaaaa agcgccaaaa    1020
   ctcgcaatat ggctttgaac cacacggtgc tgagactagt tagaatctag tcccaaacta    1080
   gcttggatag cttacctttg ccctttgcgt tgcgacaggt cttgcagggt atggttcctt    1140
   tctcaccagc tgatttagct gccttgctac cctcacggcg gatctgccat aaagagtggc    1200
   tagaggttat aaattagcac tgatcctagg tacggggctg aatgtaactt gcctttcctt    1260
   tctcatcgcg cggcaagaca ggcttgctca aattcctacc agtcacaggg gtatgcacgg    1320
   cgtacggacc acttgaacta gtcacagatt agttagcaac tagtctgcat tgaatggctg    1380
   tacttacggg ccctcgccat tgtcctgatc atttccagct tcaccctcgt tgctgcaaag    1440
   tagttagtga ctagtcaagg actagttgaa atgggagaag aaactcacga attctcgact    1500
   cccttagtat tgtggtcctt ggacttggtg ctgctatata ttagctaata cactagttag 1560
   actcacagaa acttacgcag ctcgcttgcg cttcttggta ggagtcgggg ttgggagaac 1620
   agtgccttca aacaagcctt cataccatgc tacttgacta gtcagggact agtcaccaag 1680
   taatctagat aggacttgcc tttggcctcc atcagttcct tcatagtggg aggaccattg 1740
   tgcaatgtaa actccatgcc gtgggagttc ttgtccttca agtgcttgac caatatgttt 1800
   ctgttggcag agggaacctg tcaactagtt aataactagt cagaaactat gatagcagta 1860
   gactcactgt acgcttgagg catcccttca ctcggcagta gacttcatat ggatggatat 1920
   caggcacgcc attgtcgtcc tgtggactag tcagtaacta ggcttaaagc tagtcgggtc 1980
   ggcttactat cttgaaatcc ggcagcgtaa gctccccgtc cttaactgcc tcgagatagt 2040
   gacagtactc tggggacttt cggagatcgt tatcgttatc gcgaatgctc ggcatactaa 2100
   ctgttgacta gtcttggact agtcccgagc aaaaaggatt ggaggaggag gaggaaggtg 2160
   agagtgagac aaagagcgaa ataagagctt caaaggctat ctctaagcag tatgaaggtt 2220
   aagtatctag ttcttgacta gatttaaaga gatttcgact agttatgtac ctggagtttg 2280
   gatataggaa tgtgttgtgg taacgaaatg taagggggag gaaagaaaaa gtcgtcaaga 2340
   ggtaactcta agtcggccat tcctttttgg gaggcgctaa ccataaacgg catggtcgac 2400
   ttagagttag ctcagggaat ttagggagtt atctgcgacc accgaggaac ggcggaatgc 2460
   caaagaatcc cgatggagct ctagctggcg gttgacaacc ccaccttttg gcgtttctgc 2520
   ggcgttgcag gcgggactgg atacttcgta gaaccagaaa ggcaaggcag aacgcgctca 2580
   gcaagagtgt tggaagtgat agcatgatgt gccttgttaa ctaggtacca atctgcagta 2640
   tgcttgatgt tatccaaagt gtgagagagg aaggtccaaa catacacgat tgggagaggg 2700
   cctaggtata agagtttttg agtagaacgc atgtgagccc agccatctcg aggagattaa 2760
   acacgggccg gcatttgatg gctatgttag taccccaatg gaaacggtga gagtccagtg 2820
   gtcgcagata actccctaaa ttccctgagc taactctaag tcgaccatgc cgtttatggt 2880
   tagcgcctcc caaaaaggaa tggccgactt agagttacct cttgacgact ttttctttcc 2940
   tcccccttac atttcgttac cacaacacat tcctatatcc aaactccagg tacataacta 3000
   gtcgaaatct ctttaaatct agtcaagaac tagatactta accttcatac tgcttagaga 3060
   tagcctttga agctcttatt tcgctctttg tctcactctc accttcctcc tcctcctcca 3120
   atcctttttg ctcgggacta gtccaagact agtcaacagt tagtatgccg agcattcgcg 3180
   ataacgataa cgatctccga aagtccccag agtactgtca ctatctcgag gcagttaagg 3240
   acggggagct tacgctgccg gatttcaaga tagtaagccg acccgactag ctttaagcct 3300
   agttactgac tagtccacag gacgacaatg gcgtgcctga tatccatcca tatgaagtct 3360
   actgccgagt gaagggatgc ctcaagcgta cagtgagtct actgctatca tagtttctga 3420
   ctagttatta actagttgac aggttccctc tgccaacaga aacatattgg tcaagcactt 3480
   gaaggacaag aactcccacg gcatggagtt tacattgcac aatggtcctc ccactatgaa 3540
   ggaactgatg gaggccaaag gcaagtccta tctagattac ttggtgacta gtccctgact 3600
   agtcaagtag catggtatga aggcttgttt gaaggcactg ttctcccaac cccgactcct 3660
   accaagaagc gcaagcgagc tgcgtaagtt tctgtgagtc taactagtgt attagctaat 3720
   atatagcagc accaagtcca aggaccacaa tactaaggga gtcgagaatt cgtgagtttc 3780
   ttctcccatt tcaactagtc cttgactagt cactaactac tttgcagcaa cgagggtgaa 3840
   gctggaaatg atcaggacaa tggcgagggc ccgtaagtac agccattcaa tgcagactag 3900
   ttgctaacta atctgtgact agttcaagtg gtccgtacgc cgtgcatacc cctgtgactg 3960
   gtaggaattt gagcaagcct gtcttgccgc gcgatgagaa aggaaaggca agttacattc 4020
   agccccgtac ctaggatcag tgctaattta taacctctag ccactcttta tggcagatcc 4080
   gccgtgaggg tagcaaggca gctaaatcag ctggtgagaa aggaaccata ccctgcaaga 4140
   cctgtcgcaa cgcaaagggc aaaggtaagc tatccaagct agtttgggac tagattctaa 4200
   ctagtctcag caccgtgtgg ttcaaagcca tattgcgagt tttggcgctt tttctcatcg 4260
   attgacgagg caaagggagc gagtatgcaa cctcgtaagt cagactcaga caaagaccaa 4320
   ctagttattg accagtcttc tagaaggctc tgttgtggat cttgaggccc tggagagttc 4380
   ctccaacaat ccggagacaa gcaagtcctc gtcggactag tcactaacta gactctaact 4440
   agttgcagac atggataatg caaaagagac aagcaatgaa gaaagtggta agacatttct 4500
   cctttgtggt tctggactag tctttgacta gtcacagtct taaacaagga aaatgagcat 4560
   gaaaatgagg aggaaaaggc tgctgagccc gaggaagtgc agggtgatgg cagacatggt 4620
   aggttaatac cttgttagtt attgctagtc actgactagt caataactag tctctgaaca 4680
   ccttgcaatc actccgtttg cgcagctgaa cagtggtgag gataatagta gtaagttatt 4740
   ctagcttcag agttatagga gactagatac taactagtat tagttgcaac taacctggat 4800
   ctcagagact ttggcctcaa tctagaatct atctagttgt caactagact gtggtatcat 4860
   tgtcttttat tttcctagtc ctggaactag cttctaacta gtctccctaa tatgtggctg 4920
   tcttgttttt tttttttgtt tccctacccg gatatctagt ccccttctag gttctgttaa 4980
   cctctcgggc tctgatttag tttaacgcaa acctgagatt agtttctaac tagtctctag 5040
   gttttctatc cacctttaat tgtaataata aatacaagca acgtttatac gtcaaaagca 5100
   tttataaact tttaccctaa agtagcttgc ttgtgtgttt agtttataat tagtctctta 5160
   ttaatttgat gtaggtaagc ccgccacaaa tatatatttt tacaagatac cgtggaaaaa 5220
   cttcgtgcta tcacaaaaca gtatacaaaa aataagctt 5259
<210> 2
   <211> 2400
   <212> DNA
   <213> Aspergillus nidulans
<400> 2
   aagcttattt tttgtatact gttttgtgat agcacgaagt ttttccacgg tatcttgtaa 60
   aaatatatat ttgtggcggg cttacctaca tcaaattaat aagagactaa ttataaacta 120
   aacacacaag caagctactt tagggtaaaa gtttataaat gcttttgacg tataaacgtt    180
   gcttgtattt attattacaa ttaaaggtgg atagaaaacc tagagactag ttagaaacta    240
   atctcaggtt tgcgttaaac taaatcagag cccgagaggt taacagaacc tagaagggga    300
   ctagatatcc gggtagggaa acaaaaaaaa aaaacaagac agccacatat tagggagact    360
   agttagaagc tagttccagg actaggaaaa taaaagacaa tgataccaca gtctagttga    420
   caactagata gattctagat tgaggccaaa gtctctgaga tccaggttag ttgcaactaa    480
   tactagttag tatctagtct cctataactc tgaagctaga ataacttact actattatcc    540
   tcaccactgt tcagctgcgc aaacggagtg attgcaaggt gttcagagac tagttattga    600
   ctagtcagtg actagcaata actaacaagg tattaaccta ccatgtctgc catcaccctg    660
   cacttcctcg ggctcagcag ccttttcctc ctcattttca tgctcatttt ccttgtttaa    720
   gactgtgact agtcaaagac tagtccagaa ccacaaagga gaaatgtctt accactttct    780
   tcattgcttg tctcttttgc attatccatg tctgcaacta gttagagtct agttagtgac    840
   tagtccgacg aggacttgct tgtctccgga ttgttggagg aactctccag ggcctcaaga    900
   tccacaacag agccttctag aagactggtc aataactagt tggtctttgt ctgagtctga    960
   cttacgaggt tgcatactcg ctccctttgc ctcgtcaatc gatgagaaaa agcgccaaaa   1020
   ctcgcaatat ggctttgaac cacacggtgc tgagactagt tagaatctag tcccaaacta   1080
   gcttggatag cttacctttg ccctttgcgt tgcgacaggt cttgcagggt atggttcctt   1140
   tctcaccagc tgatttagct gccttgctac cctcacggcg gatctgccat aaagagtggc   1200
   tagaggttat aaattagcac tgatcctagg tacggggctg aatgtaactt gcctttcctt   1260
   tctcatcgcg cggcaagaca ggcttgctca aattcctacc agtcacaggg gtatgcacgg   1320
   cgtacggacc acttgaacta gtcacagatt agttagcaac tagtctgcat tgaatggctg   1380
   tacttacggg ccctcgccat tgtcctgatc atttccagct tcaccctcgt tgctgcaaag   1440
   tagttagtga ctagtcaagg actagttgaa atgggagaag aaactcacga attctcgact   1500
   cccttagtat tgtggtcctt ggacttggtg ctgctatata ttagctaata cactagttag   1560
   actcacagaa acttacgcag ctcgcttgcg cttcttggta ggagtcgggg ttgggagaac   1620
   agtgccttca aacaagcctt cataccatgc tacttgacta gtcagggact agtcaccaag   1680
   taatctagat aggacttgcc tttggcctcc atcagttcct tcatagtggg aggaccattg   1740
   tgcaatgtaa actccatgcc gtgggagttc ttgtccttca agtgcttgac caatatgttt   1800
   ctgttggcag agggaacctg tcaactagtt aataactagt cagaaactat gatagcagta   1860
   gactcactgt acgcttgagg catcccttca ctcggcagta gacttcatat ggatggatat   1920
   caggcacgcc attgtcgtcc tgtggactag tcagtaacta ggcttaaagc tagtcgggtc   1980
   ggcttactat cttgaaatcc ggcagcgtaa gctccccgtc cttaactgcc tcgagatagt   2040
   gacagtactc tggggacttt cggagatcgt tatcgttatc gcgaatgctc ggcatactaa   2100
   ctgttgacta gtcttggact agtcccgagc aaaaaggatt ggaggaggag gaggaaggtg   2160
   agagtgagac aaagagcgaa ataagagctt caaaggctat ctctaagcag tatgaaggtt   2220
   aagtatctag ttcttgacta gatttaaaga gatttcgact agttatgtac ctggagtttg 2280
   gatataggaa tgtgttgtgg taacgaaatg taagggggag gaaagaaaaa gtcgtcaaga 2340
   ggtaactcta agtcggccat tcctttttgg gaggcgctaa ccataaacgg catggtcgac 2400
<210> 3
   <211> 10
   <212> DNA
   <213> Artificial
<220>
   <223> crippled Kozak sequence
<220>
   <221> misc_feature
   <222> (1)..(1)
   <223> n is a, c, g, or t
<220>
   <221> misc_feature
   <222> (3)..(4)
   <223> n is a, c, g, or t
<220>
   <221> misc_feature
   <222> (9)..(10)
   <223> n is a, c, g, or t
<400> 3
   nynnatgynn 10
<210> 4
   <211> 45
   <212> DNA
   <213> Artificial
<220>
   <223> PCR primer
<400> 4
   tgattacgcc aagcttgcgg ccgcgaccaa agcgtgcgaa tagcg 45
<210> 5
   <211> 37
   <212> DNA
   <213> Artificial
<220>
   <223> PCR primer
<400> 5
   tgcagctgat aagcttggtg actataaaca atcgccg 37
<210> 6
   <211> 44
   <212> DNA
   <213> Artificial
<220>
   <223> PCR primer
<400> 6
   gacggccagt gaattcgcgg ccgccgagga actctgctac tgcc 44
<210> 7
   <211> 37
   <212> DNA
   <213> Artificial
<220>
   <223> PCR primer
<400> 7
   taccgagctc gaattcgggc cgacgagttg gaaaagc 37
<210> 8
   <211> 21
   <212> DNA
   <213> Artificial
<220>
   <223> PCR primer
<400> 8
   ggagtccgta tagtaagcat c 21
<210> 9
   <211> 28
   <212> DNA
   <213> Artificial
<220>
   <223> PCR primer
<400> 9
   tgtacagcac tgtattggta tgtgaacg 28
<210> 10
   <211> 20
   <212> DNA
   <213> Artificial
<220>
   <223> PCR primer
<400> 10
   catgtagata agattagggc 20
<210> 11
   <211> 60
   <212> DNA
   <213> Artificial
<220>
   <223> PCR primer
<400> 11
   gacgaattct ctagaagatc tctcgaggag ctcaagcttc tgtacagtga ccggtgactc 60
<210> 12
   <211> 27
   <212> DNA
   <213> Artificial
<220>
   <223> PCR primer
<400> 12
   gacgaattcc gatgaatgtg tgtcctg 27
<210> 13
   <211> 4664
   <212> DNA
   <213> Aspergillus oryzae
<400> 13
   agatctagga aaggaggcag cttgggctga gtattccagc gcaaaattct tattcttagg 60
   ctaccttgtg gaaattgcaa gtaaccggga tatccatttg gtgattatgg tacagggaga 120
   gaaaacgcaa aaagtggttg agcgatacct gataggcaaa ggtctcatat atactcgacc 180
   tcgtgaggaa atggggtctg gcaccaatct ggaggtttct ctggtaaaag gttccttgag 240
   tttggggatc caatctacac tcagtgaggg gatcactgag acatacaaat cgccctctgc 300
   aatcattgcc ctggactcgt ctttaaatgt gaagagccct tcagttgaac atatgcgaac 360
   cacatttgct cgtcatggta acctacttcc catcattcga ctcattgtct caaactccag 420
   tgagcacatc gaactctgtt tcccggatcc tccagagctc caacgtcttc agttgatcgt 480
   ccaatacact gtccgtctcc gcaatatagt aggtgatctg caggacgatg cccttggtgt 540
   gcgtgaagac gtggaagaga tattgccttg gctttattca gatcacttca gcattagctg 600
   gcctctgact cccattgaac ctttacacgt cgtgagctcc gataagctgt tatctgttca 660
   acttgaagct caaccccaaa caactgttgc tggtacacca aaccacaaca cacaagctca 720
   aaagcgtcta tttgtggaag attcaagcga gcatacgtct aaaagactac gggtggaatc 780
   gtcacaggat aacactcaac ttacagagtc aacaaaattt ccaagtcaaa ccttggatag 840
   cggtctgcat gccttagaga agaatctcgt gcagatgaga actacgcatg ccgctgagct 900
   tgagaagttc cagaatgcat taactgacat gcaaacccgc ttacaggaga gagagaaact 960
   actcgaatcg ctccagcatc gctatgaaac tcgaactaaa gacctccaca agatccggcg 1020
   ggagcgggat cgcttggctg agtataaagc cacgtcagag cagaaaattg aaaaacaaag 1080
   agaagatatt agcaagctaa aggatgagcg cacccagcta aggcaggatc tcgagcaggc 1140
   aagggcggag atcaaaactg gaggaggtgc tgtagcagag ctggagacgg ctcgggaaga 1200
   tattcgacgc ctgacacaag aaaatgctgg cctggaacgg aaggcagaat acgaggccaa 1260
   gcaagccgag tacactcgcg agcagtacca aactgcgtct aatatggcgg ctcaaactgg 1320
   gaacgaggtc cgccaacttc gagaagagaa cgagttatta aagcgtaagg ttgctgggaa 1380
   tgctagtcgt ctacgagaga tcaataaaga gaacgacggg gcacgacatc tatctcgtat 1440
   ctctgaactg gaagcttctc ttgcatcccg cgaagacctg ttacgccgaa aagaggatga 1500
   gctgcgggaa attcgcaaga accgaccctc cacccgctcg accagtactc aacctcgcag 1560
   cccgagactg accgctggaa gtcgtccaac gagcccagga atcaataatc ataacggacg 1620
   tggcagcgcg ttaaggttca gctctgagat gcccagttaa gggtcttacc tgttttgcat 1680
   aacttcacca gtttaagtgg aatttctatc atgttgcagc gactccaccg atttcattct 1740
   tgcgactatg acgtttacat agaggagggc gtgcatttga tgtttttttt ttcatatcaa 1800
   tgactttgca ttgtctgggc gttttgtatg cctgcaccct ttttctcatt ttattatctc 1860
   attttgtgtg tggatcctgc gccccttctc aagttttcta cgttagattt tgaattggat 1920
   gccataacca gtgtacgtca tccttatgac tacattgctc aatctgtttg caaatttttc 1980
   tactgttcta ctgcccgagg acttcgtttt ccacatccaa tattagcgat gtttcaatat 2040
   gtaggctgtt ttcgtattga ttatgctacg tgacaatatc tccttgttca aatgtataat 2100
   tgattaatta cgtacaatta gtgcaagccg ttacttactg aatgatagtg ctgtaatccc 2160
   tggttattca aaacatgaga tcaacgacat gaacaagcca tatgaacgat agggaaaata 2220
   agtgcaatat aatgatagaa aataacataa taagcttcat cagtctttaa gccatggcgt 2280
   tagccataaa tgtagacaac tctttcgctt tttcgccctt cgtcatcgta aaatgattag 2340
   catcttccat tactgtgatt ccaccaatat tctcaggtcc aaccaatgta tcccacttgt 2400
   taggtcccag atcggtccgg tcattaagaa gccagaccat ctccttggga tccttggaac 2460
   cgtctgtcgg gggatcaggc cggggatctc ccggcttacc gcaaacaccg tccttggccc 2520
   agatgatata agtcttgggc agtttcttgg cccatttctc gtctttgaat ggaaggggcg 2580
   cggccttgta tgcgtcgaga gcgtcgataa aggctaggaa gtggggtagg agccaggagg 2640
   gtggtgccgt tttaccttca ccgaagagac cgatagagtt gaagaagccg tacaatctct 2700
   gcggcagctt ctcgaggccg atggggaagg gggtatcaag gagaagcaag cggtcgactt 2760
   gttcaccttc ctcaaacatt agatggcgtg ccgcatcata tgcacagatc cctcctgctg 2820
   accagccacc gaagctgtaa ggacccttgg gttgccgacg acgaatctct gctacatagg 2880
   gcgcagtgag ttcatctagg ctgcatttga gcttctcagg tgtcttcata tatgggcaat 2940
   tcaacccgta aacgcagacg tcaggagaga gtccggggat ggtagcgtaa gatgtagctg 3000
   agccagagcc gtcaggaaac aagaataacg tctttgttgc tgtcctgggg tttccttgta 3060
   acaggatgga tgtggccttg ggatgttgag caagctttgg ctggaggtcg tggatagctt 3120
   cagggaattt cactggctct ggcataggaa ttggagcaga ggtagtcttg ggttttaggt 3180
   ccaaagccac ctctatatca ttgagggtct ggttttcgat gaagaactct cctggcaggt 3240
   ccatgtccaa agtctcccgt attcttccaa gaacagtaag ggagagaagc gagtccatac 3300
   ccatctctcc caagttctcg tcagggtcga tctcttctgc gggtacaccg atctcctccg 3360
   ccatgatgtt acggatttcc ttcatggtgc cgttttgctc gacttgagct accttctcat 3420
   tcggcggtga cgttatgtca ggtgtgcttg cgcttgagga ctcgccaccg ttgaaagaga 3480
   aattcgactc ttgttcactg gaggaaccat catggctctc tgaaggactc acttgggcga 3540
   tgagccgctt gaagtcgttg acagttggat gatcggtaaa cacagaggag tccaaatcga 3600
   ggttcaactc ttcacgatac ctgccagtaa ctgtaaggga gaggagtgag tctacaccgt 3660
   agtccgcaaa caccatgtcg tctgagattt cagactctgc caggccaact tctgaggcca 3720
   gaatgctgag tgcgcgtacg acaacactcg gcccagaaga tttggtgaac gacttggaag 3780
   gaacaggtgc tttaggacgg gtcttggcgc cggtgttggc cttctgagtg cgagctggag     3840
   gtggtgattt cgctgtaatt ggtgcctttg aaccgccaac agggggaaga acagtgtcaa     3900
   gtatcttgcg tgccagtgct tggaactaat tatcatgtca gtaagcagga accaaaatca     3960
   gtagtgaggt acccaccttc acacctccga agacagcaat aatatcgtcg ccctcaaaga     4020
   tataaacatc tccagcccaa atgttgtttt gccatggctg catcctcaca taagtgcgat     4080
   aagtgacatc aggcgagaac ttcttcaggc aacgcatcga atcccatcca tggttaacaa     4140
   atacttggtt cttagagtcg gtcgcgtcgc tcgcattcat aatgaatccg gacaagtgac     4200
   cgaaactgtc aatccagaat gggtttcggt ggaaatttcc tggcggtgct tggaatttga     4260
   cacgagcggt ggcctcatta ttgtcgctgt ccaggataac ctcgcgaatc gacttgaaat     4320
   tttcatcata atcgaccaac gcgctgaaaa gtttatagac cattcctcgc tggatacggt     4380
   gagcctcccc tgtctccgca ttctggtgaa gactgtcgat gcttctcttg accaggtacg     4440
   agattctctt ccactcgagc tcggaggcat tggtatcaaa gaacttcacc gtacaatacg     4500
   catgatccac ggtctttttg ccgtcagcat taacggcgta tacttgaact gaagccttct     4560
   tctcagccca attagcaata gcagagactc taaagagctg ctctcctccc ttcgcgatga     4620
   gtggcttcgg tacgaccatg tcacacacat cgagaccaag atct                     4664
<210> 14
   <211> 2249
   <212> DNA
   <213> Aspergillus oryzae
<400> 14
   atggctgacg aggatcaata tcgtggagac gaccagatcg atgaggaaga ggaggagacc       60
   gacgagagtg tacacacttt caaacacacc tgaaagcttc ggaggctaac atgttatcaa      120
   ccaaaatagg gatacaaaac agtgaaagat gccgttcttt ttgctatcga agtcagcgat      180
   tcgatgctca cgcctcgtcc atcttccgat tcaaagaaac ctgcggagga gtcccccaca      240
   acggccgcac taaaatgcgc atattatctc atgcaacaac gcattatctc taatccccgt      300
   gacatgatcg gtgtgctatt atatgggacg caggcgtcca aattttatga cgaggatgaa      360
   aatagtcgag gagatctttc atacccacac tgctaccttt tcacagacct tgatgtcccc      420
   tctgcgcaag aagtcaagaa tcttcgggca ctagcacaag acggcgatga atcaaaggat      480
   gtacttaagg cgtcaggcga gcgggtctca atggcgaacg tactcttttg cgccaatcaa      540
   atattcacgt cgaaagcccc taacttcttg tctcggcgat tgtttatagt caccgataat      600
   gatgaccctc atggcgataa taaaagcttg agatccgctt caactgtacg cgcgaaggac      660
   ttatatgacc tcggtgtcac tattgagctg tttccgattt ctcggccagg ccatgagttc      720
   gataccgcca gattctatga cgtaagatta tattgactca atgtgaagta tcgctgctaa      780
   cagcaattag gatatcatct acaaggcctc tccttcggat ccagatgccc cggcatacct      840
   gcaaaccgat tccaaggctt ctccagccac cggggatggg atatcactgc tcaataccct      900
   cctgtccaat atcaattcaa gatctgtccc acggcgtgca cagttctcca atataccatt      960
   ggagcttgga ccaaacttaa aaatatctgt ctcaggatat cttttgttca agcgtcaagc 1020
   acccgccaga aactccttca tctggctcgg cggtgaacag ccccagattg tcaaaggagt 1080
   gaccactcaa atcgctgacg acacggctcg cacgattgaa aagtgggaaa ttaagaaagc 1140
   ttataagttt ggcggtgatc aggttgcttt cacgcccgaa gagatgaagt cactgaggaa 1200
   cttcggtgat cctgtcatcc gtataatagg gttcaagccc ctctctgcac ttccgttctg 1260
   ggccaatatc aaacacccct cctttatata cccatcggaa gaagattttg tgggctccac 1320
   gcgggttttt tctgctttgc atcagacact cctccgggat aaaaaggccg cacttgtctg 1380
   gttcattgct cgtaaaaatg caagtcctgt tctgggggct atggtcgccg gagaagagaa 1440
   actagacgag agtggcgtcc agaagtttcc tccaggaatg tggataatac ctctcccgtt 1500
   cgctgatgac gtccgtcaaa accctgaaac cacactccat gttgcacctg agccattgat 1560
   cgatcaaatg cggtatattg tccagcaatt gcaacttcca aaggcgtctt acgacccctt 1620
   taagtaccct aatccatgta agcttctgcc aacttcctgc acagaaactc tggcattaac 1680
   ctattgctct gttagccctc caatggcatt atcgcattct acaagccttg gcgttggatg 1740
   aggacctccc ggagaagcca gaagacaaaa cgttgcccag atatcggcag atcgataaag 1800
   tatatcacac attcctattc tttccacgga tcttgctgac cttcgcttag cgcactggcg 1860
   actatgtatt gtcttgggcc gacgagttgg aaaagcaata cgcgaaaata tcggcacatg 1920
   gcccgaagag cacactcgtc aaacgaagcg ccaaagaccg aacatctgaa gtcgaggatg 1980
   cagcccagaa gccatacaag aaagtgaagg tggagacaga cgagcaaggc gttgaagatg 2040
   tagtgcgagc ccattaccag aagggatcgc tatcgaaggt gactattacc tgccccctag 2100
   gctttaattt ggactaacta acgcgcgtga cttgtgtgta gcttacggta cctgtcctca 2160
   aagactttct gaatgcccat ggacgctccg ctgctgggaa gaaagctgat ctcgttgagc 2220
   gtgtggagga gtatttggag cagaaatga 2249
<210> 15
   <211> 2662
   <212> DNA
   <213> Aspergillus oryzae
<400> 15
   atggcggaca aggaagcaac tgtgtatatt gtggacgttg ggaggtccat gggagaatgt 60
   cgcaatggcc gatcagtgac tgatcttgaa tgggccatgc agtatgtctg ggatcgcatt 120
   acaggaacag tgagtggcag tcgtcacaat tggaccgtat tcgttaaata ccttgctcaa 180
   tttcaaacca ggtggccact ggccgcaaaa ctgccatgat gggtgtgatt ggactcagga 240
   cagatggtat gtatacttct gaatactgta tgcggttcat acgctgatcc aaaaattaga 300
   aacgtccaac gaacttgaag atgacgtaca tttctctcac attgcagttc tgtcgaacct 360
   caaacagtat gctttccact ctatgataat ttggtttgtg cgccaaactg acgaggacgt 420
   caaggtttct tatgccggac attcggaaac tggaagatga actgaaaccg agcaaaacgg 480
   acaaaggaga cggtaagctt tttgagagcc actaggacct actgtccaat ttactaaact 540
   ttgttctcta gctatttccg ctattatctt ggctattcag atgattatca cgcattgcaa 600
   gaagttgaag tacaggcgca agatcgtcct cgtcactaac ggacaggggc gcatgagcga 660
   tgaagacctg ggcgagattg tgaagaaggt caaggaagat aacatcgagc ttgttgttat 720
   gtcagtgatt tgctaccaga tagcaacgaa acaaaagcta acttcaagca ggggaattga 780
   tttcgatgac cctgagtacg gttacaaaga agaagacaaa gaccctcaca aggtagcgat 840
   atctctcgcg cagctttgtt cttttctaac aactaaaaca ggccgaaaat gaaactctct 900
   tgcgtaccct tgtggaagat tgtgatggag tctatggaac attcgagcag gctgtggctg 960
   aactagacat cccccgtgtc aagtctgtca ggtcagtggc aagcttcaaa ggatatctcc 1020
   aactaggcaa cccagaggag tatgactctg ctctccgcat tcctgttgaa aggtactatc 1080
   ggacttaccc ggccaaaccc ccgaccgcaa gttctttcgt cctgcgctca gagcctgaag 1140
   ctggacaaga agaggcagag tcatctgagg ctgctgctgc tacgcaaaaa gggagccaat 1200
   ctggagatgc cggattgacc actgtgagaa ccatgagaac atatcaagtt gaggacaaaa 1260
   gtgcaccggg tgggaaaatc gacatcgaac gagatgagct cgccaaagga tatgagtatg 1320
   gacggacagc agttcacatt agtgaaactg acgagaacat cacgattctc gatacattcg 1380
   cagggctgga gttgatgggc ttcatccaga ctgaccaggt atgtcttgct gaagtcgcct 1440
   cggtgcatgc tctgacacac gattatagta tcaacgttat atgcacatgt ccaacacaaa 1500
   catcataatt gcacaacgtg ccaacgacaa agcagctctt gccctttcat cctttataca 1560
   cgcccttttt gagctagaat gctatgctgt tgctcgccta gttgtgaaag agaacaagcc 1620
   accagttata gtcttgctcg cgccctcgat cgagcctgag tatgaatgcc ttctcgaagt 1680
   ccagttacca tttgcggaag atgtccgaac ctatcggttc cctcctctgg ataaagtgat 1740
   tactgtttcc ggaaaggttg tgacacaaca ccggaatctt cccagtgatg atttactcga 1800
   tgtgatgggc aagtacgtga atagtatgga gcttgtcgac gcagatgagg atgggtaggt 1860
   ttatgcctaa aagattccga atatcttctc attgacataa ccagggatcc agtggagact 1920
   ttccctatcg acgactcgta ttccccagtt ttgcaccgga ttgacgccgc catccgtgct 1980
   cgggctatac atcctgacca gcccatacct cctccatcag agagactgac aaaattctca 2040
   cacccacgag aggatctcat cgagaaatca cagaaacacc tagagaagtt gatcgagata 2100
   gccgatgtta agaagggttg gacatcaccc cacaatcaag tctgtcagac tgctaattca 2160
   gttaccagtt cctcccaaag cgaagggtcg caagcgcact cgtgaaaccg aaaagccact 2220
   ttccggactc gacgtcgatg ccctgcttca tcatgaaaag cgcgtcaaga tatctcccaa 2280
   caatgccatt cctgagttca agcagactct cgcacaggcc gagaatatcg aggccatcaa 2340
   agacgctaca aagcagatga tggtcatcgt tgaagatcaa atcaaacaca gtctcggtaa 2400
   tgctaactac gaccgggtca ttgaagcgct gggcacgatg cgtgacgagt tggtatctta 2460
   cgaagagcct gcctcctaca atgacttcct gggccagctc aaggataagt tactgcagga 2520
   gaagcttgga ggagaccgac aagagctgtg gtggcttgtt cgacgaaaca agctgggact 2580
   tgtcactcag cgcgagtcgg atcaatctag ggttaccgat acggaagcca aagaagtaag 2640
   tctcactaag atgaaggagt ga 2662
<210> 16
   <211> 2284
   <212> DNA
   <213> Aspergillus niger
<400> 16
   atggcggacg gcaacccaca tcgggaagat gaggcggccg aggaagaaga ggagattgat      60
   gagactgtac gcaaatttac ccatgaactt ggactggaac tctggaactg acaataagat     120
   cagagctaca aaccagtcaa agatgcggtc ctcttcgcaa tcgatgtcag cgattccatg     180
   ttgacgcctc gcccctcagc agatcctaag aaacacaccc aagaatcacc caccacggca     240
   gcgctcaaat gcgcctatca cttcatgcaa caacgaatca tatcaaatcc acaagacatg     300
   atgggtgttt tgctgttcgg gacccaggcg tccaagttct ttgaagaaga tgaagacagt     360
   cggggagacc tgtcctaccc caactgctac ctcttcactg atctggatgt tccttcggct     420
   catgaggtca aggacttcgg agcactggta gatgatgaag gagactcaag ggaggttcta     480
   tctccagcga aagagcaggt ctctatggca aacgtcctat tttgcgccaa ccagatattc     540
   acatccagag cgccaaattt cctctcccgg cgtttgttca tcataaccga caatgacaac     600
   ccccatggtg atgataaaac cctgcggtca gcggcgactg tacgtgctaa ggatctttac     660
   gatcttggtg tcacaattga gctgtttccg atctcacgcc ctgagcatga gttcaagaac     720
   agcaagttct atgacgtaag ctatcatact ctatagcaaa gtggcagggg tcgatactca     780
   ctacagatac aaaggatatt atctacaagt cattgcccag cgatccagag gcgcctgcat     840
   atctacaatc tgattcaaaa gcggcgactg cgaccgggga cgggatttca ctcctcaaca     900
   cgcttctgtc cagtattaat tcgagaacgg ttccgcgtcg cactcatttt tcgaacatgc     960
   ctttagaact tggcccagac ttcagaattt cggtatcggg ctatatactc ttacgaaggc    1020
   aagcgcccgc tagaaactcc ttcatctggc tgaacggcga gaagcctgtg gtcgcgaaag    1080
   gagtgacttc ccactccgca gatgatactg gccggactgt cgagaaatgg gagatcagaa    1140
   aggcatataa gttcggtggc gaccaagtaa ccttttcgcc tgatgagcag aaggcgctta    1200
   gggatttcgg tgagccagta atccgggtta ttgggttcaa gcctatcact gcgcttccat    1260
   tctgggcaaa cgtcaagcac ccatatttta tctatccatc cgaggaagac tatgtaggct    1320
   cctcgcgagt attttccgca ttgcatcaga ctcttttgcg ttccaagaag atggcactcg    1380
   tctggttcat tgcacgcaag ggtgctggcc ccgttctcgc cgctatgatc gcaggcgaag    1440
   aaaagcttga tgagaatggc gtacaaaaat accctcctgg catgtggatt cttcccctcc    1500
   ccttcgcaga cgatatccgg cagaaccccg aaacaacgtt gaatgtcgcc ccggagtcat    1560
   tgattgatca gatgcgcgtg atcgtccagc aactgcagct gccgaaggga gtgtacgagc    1620
   ctctcaaata ccccaatcca tgtaagtcac ttctgtcttg cattgctcgt atacgatgaa    1680
   cgagaagctg acagcccgtg atcagccctt caatggcatt accgcatcct acaagctctc    1740
   gcattagacg aagatctccc cgaaaaacca gaagacaaaa ccattccgaa ataccgccaa    1800
   atcgacaagg taaatccacc acacccaaca cgagaaataa ccctccaggc gtccaactta    1860
   ctgacaattg caccacagcg cgccggtgac tacgtattat cctgggccga cgaactcgaa    1920
   aagcaatacg ccaaaacctc agcagcggcc cctcgcccaa ccagcaccct cgtgaaacga    1980
   ggatcaaaag accgagcaag cgaaaccgag gactccaagc catcgaaaaa gatcaaggtt    2040
   gaggaagact ctggaagcct agaggaggaa gtccgcaggc atcacaagaa gggaacgcta    2100
   tccaaggtaa gccaccacag gctttctaca cgtcctcgtg atggcaaata tgacatcgta    2160
   ttaaccggcg gttttctagc ttacggtcgc tatcctcaag gacttcttga cttccaatgg    2220
   acgctcaaat gccggtaaga aggcggatct tattgagcgg gtagaggagt tcttggagca    2280
   gtga                                                                 2284
<210> 17
   <211> 2651
   <212> DNA
   <213> Aspergillus niger
<400> 17
   atggccgata aagaggcaac tgtctacatc gtggactgcg gcaagtccat gggggagcgg      60
   cgtcatggtc gcgaagtgac ggatctcgac tgggcgatgc aatatgtttg ggatcgtatt     120
   acagggacgg tgagatcctt attcttgaga atcatatcat acatgaaagc ttatgttttg     180
   gataggtggc cactggacga aaaatggctt tgatcggtgt tcttgggctc aggacagatg     240
   gtgagtgact agcctcccgg gtacagttgg tagttgtagt ttgctggtcg gggctaatgc     300
   aggaacgtcc agaaaccgct aatgagttgg aggatgatcc tgattattcg catatctcgg     360
   ttttgtctgg gattaaacag tatgattcat ttttgtctgc tgatcctctg gttattcgct     420
   gatgaactat aggtttctta tgccggatat ccggggtttg agcgaccgaa taaagcctag     480
   caagactaat aagggagatg gtgagttact cttcttgtat ggaattggag tgattggggc     540
   tgagccgatg aatatagcta tctctgcact tgtgctcgcg attcagatga ttatcactca     600
   gtgcaagaaa ctgaagtaca agcgcaggat tgtcctggtt actaatgggc agggcccgat     660
   gaacccggat aatcttagtg aaataacgaa gaagattaag gaggataaca ttgaacttat     720
   tattctgtta gtgtcaattg atacactgag agaaccgggg tactaacatg ctgcagggga     780
   ccagactttg atgatcctga atatggggtg aaagaggaag ataaagatcc gcgaaaggta     840
   tttaacttcg ttccatatgc tctagactaa taataacaat ggctacaggc cgaaaatgaa     900
   acactcctgc gtagtcttgc cgaagactgc gaaggagcct atggaaccct agaacaagct     960
   gttgcggagc tggaaactcc tcgtgtgaaa accacaagga taacagcaag cttcaagggc    1020
   catttgcaac taggaaaccc cgcagaatat gatactgcag ttcggatccc tgtggagcgc    1080
   tactacagga catacgttgc aaaagctccg tcggctagtc agttcacagt acgtaacgaa    1140
   gaggagatgg gaatggccgc ggccgcagcc ggctcgcagg aaggtagttc ccttgtgggt    1200
   gttcgaaaca acaggtccta ccaaattgac gatgggacta ctgaagaagg ggtgagggac    1260
   gtggatcgag agcaacttgc caagggttat gagtacgggc ggacattggt ccctattagc    1320
   gagacggatg agaatatcac caccctagag acatttgcgg ctatcgagct tcttgggttt     1380
   atacagagcg atcgggtgag ttctaccctc caataactgt tattatgctg ctaagtgggt     1440
   tttgccatta gtatgatcga tacatgcaca tgtcgacgac aaacatcatc atcgcgcagc     1500
   gcgcgaatga caaggcagca ctcgctcttt cctctttcat acatgcgctg ttcgagctgg     1560
   aatcgtacgc tgtcgcccgt atggtgctaa aggagaacaa accccctgtc atagtcgtgc     1620
   ttgcgccatc aatcgaaccc gactacgagt gtctcctcga agcgcagttg ccattcgcag     1680
   aagacgtacg aacgtaccgc ttccctccac tcgacagagt cattacagtg tctggtaaag     1740
   tggtgacaca gcatcgaaac ctacccaacg acgatctgtt gaatgcgatg gacaaatacg     1800
   tgaaaagcat ggagcttacc gatatggacg agaacgggtg agaagaattg gaagtgatct     1860
   caacttcact gctgactttg tacaaagtga cccgacggaa tctctcccaa tagacgactc     1920
   tttctctcca gtcctgcacc ggatcgactc cgcaatccgt caccgtgcca ttcatcccaa     1980
   cgaccctatc ccgcccccag cctcagtcct aacgaagttc tcccaccctc cggatgacct     2040
   cgtcgagaag tccaagaaat acctagacaa gctagtagca gtgtcggacg tcaagaaagg     2100
   tcagtccatc tcggccttga gcctcttagg cccccatcat actcacagtg atgaatctag     2160
   tcccaccaaa aaccaaaggc accaaacgga cccgcgaaac cgagaagcca ctatccggtc     2220
   tcgacgtcga tgcccttctc caccaagaga agcgcacgaa gatctcaccc aacaacgcaa     2280
   ttcccgagtt taagcagacg ctctcgcagg cagagaacat cgagatcatc aaggatgcag     2340
   tgaagcagat gagcactatc attgaagacc aaatcaggca tagtcttggc gatgttaatt     2400
   atcatcgggt cactgagggg ctaggtgtga tgcgggagga actgatcgat tatgaggaac     2460
   ctgctctgta taacgatttc ttgaagcagc tgaaggagaa gttgttgaaa gaggagctcg     2520
   gtggggatcg acgggagctg tggtggctgc taagaaggag taagttgggg ttgattgaac     2580
   agagggagtc ggaacactct gaggtgagag aagaggaagc gaaggcgttt atgtctatgg     2640
   ctgctaagtg a                                                          2651